(19) **Europäisches Patentamt / European Patent Office / Office européen des brevets**

(11) **EP 4 682 172 A1**

(12) **EUROPEAN PATENT APPLICATION**
published in accordance with Art. 153(4) EPC

(43) Date of publication:
21.01.2026 Bulletin 2026/04

(21) Application number: 24770009.9

(22) Date of filing: 14.03.2024

(51) International Patent Classification (IPC):
$C07K\ 16/30$ (2006.01)    $C12N\ 15/13$ (2006.01)
$C07K\ 16/18$ (2006.01)    $A61K\ 39/395$ (2006.01)
$A61P\ 35/00$ (2006.01)

(52) Cooperative Patent Classification (CPC):
A61K 39/395; A61P 35/00; C07K 16/00;
C07K 16/18; C07K 16/30; C07K 16/46

(86) International application number:
PCT/CN2024/081748

(87) International publication number:
WO 2024/188319 (19.09.2024 Gazette 2024/38)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 15.03.2023 CN 202310261481

(71) Applicant: Sunshine Guojian Pharmaceutical
(Shanghai) Co., Ltd.
Shanghai 201203 (CN)

(72) Inventors:
• ZHAO, Jie
  Shanghai 201203 (CN)
• JIANG, Liangfeng
  Shanghai 201203 (CN)

• ZHENG, Zhong
  Shanghai 201203 (CN)
• ZHU, Zhenping
  Shanghai 201203 (CN)
• ZHANG, Hao
  Shanghai 201203 (CN)
• LOU, Jing
  Shanghai 201203 (CN)

(74) Representative: dompatent
Partnerschaft von
Patentanwälten und Rechtsanwälten mbB
Deichmannhaus am Dom
Bahnhofsvorplatz 1
50667 Köln (DE)

Remarks:
The complete document including Reference
Table(s) and the Sequence Listing(s) can be
downloaded from the EPO website

(54) **ANTI-MUC17, ANTI-CD3 AND ANTI-CD28 TRISPECIFIC ANTIBODY**

(57) Provided is an anti-MUC17, anti-CD3 and anti-CD28 trispecific antibody. Specifically, the trispecific antibody targets tumor specific antigen MUC17, and also targets CD3 and costimulatory molecules CD28 expressed by T cells so as to provide a costimulatory signal for T cells, thus stimulating stronger and more effective killing activity. Therefore, the trispecific antibody is expected to effectively overcome the drug resistance of solid tumors to T-cell engagers.

EP 4 682 172 A1

# EP 4 682 172 A1

**Description**

**TECHNICAL FIELD**

The present invention relates to the field of antibodies, and in particular to an anti-MUC17*CD3*CD28 trispecific antibody.

**BACKGROUND ART**

According to the data in the World Cancer Report 2020 released by the International Agency for Research on Cancer (IARC) under the World Health Organization (WHO), there were 19.29 million new cases of cancer worldwide in 2020. China alone accounted for 4.57 million new cases, representing 23.7% of the global total, significantly surpassing other countries. In 2020, the top 10 most prevalent cancers in China were: lung cancer, breast cancer, gastric cancer, colorectal cancer, liver cancer, esophageal cancer, cervical cancer, thyroid cancer, uterine cancer, and prostate cancer. Lung cancer had the highest incidence rate, followed by colorectal cancer and gastric cancer, accounting for 12.3% and 10.5%, respectively. Therefore, identifying and exploring therapeutic targets related to gastrointestinal tumors for developing more effective clinical treatments holds significant clinical importance.

MUC17 is a high-molecular-weight O-glycosylated mucin that belongs to the SEA family of transmembrane mucins, along with several other mucins (such as MUC1, 3, 12, 13, and 16). Data from clinical cancer studies indicate that MUC17 is highly expressed in a variety of gastrointestinal tumors, including gastric and colorectal cancers, making MUC17 a potential target related to gastrointestinal tumors.

Bispecific T-cell engagers (BiTEs) are bispecific antibodies formed by the fusion of fragments from two distinct antibodies. The BiTEs can bind CD3 molecules on the surface of T cells as well as antigenic molecules on the surface of tumor cells, respectively, thereby inducing the killing of tumor cells by T cells, and this process is not restricted by MHC. This therapy is currently more effective in hematologic malignancies, and a CD3 x CD19 BiTE, Blinatumomab, for such a therapy has been approved by the FDA for the treatment of B-cell malignancies. Due to the physical barriers and immunosuppressive microenvironment present in solid tumors, BiTEs have not achieved the expected efficacy in the clinical treatment of various solid tumors. Therefore, there is an urgent need in the art to provide a novel T-cell engager capable of overcoming the drawbacks of BiTEs, such as a trispecific T-cell engager (TriTE) targeting the tumor-specific antigen MUC17.

**SUMMARY OF THE INVENTION**

An object of the present invention is to provide an anti-MUC17*CD3*CD28 trispecific antibody.

Another object of the present invention is to provide a trispecific T-cell engager (TriTE) that targets the tumor-specific antigen MUC17 and also targets T-cell expressed CD3 and costimulatory molecules CD28.

In a first aspect of the present invention, there is provided a multispecific T-cell engager, comprising:

a first targeting domain D1, wherein the D1 binds to a target protein selected from the group consisting of: CD3, CD28, CD40, CD137;
an optional second targeting domain D2, wherein the D2 binds to a target protein selected from the group consisting of: CD3, CD28, CD40, CD137; and
a third targeting domain D3, wherein the D3 comprises one or more MUC17 antigen-binding domains.

In another preferred embodiment, the D1, D2 or D3 is each independently selected from: single-domain antibody (sdAb), Fab, Fab', F(ab')$_2$, TriFab, Fv fragment, fragment variable Fv heterodimer, single-chain Fv (scFv) fragment, diabody, bispecific T-cell engager (BiTE), or single-domain fragment, preferably single-chain Fv (scFv), Fv fragment, or Fab fragment.

In another preferred embodiment, the MUC17 antigen-binding domain comprises a third heavy chain variable region and a third light chain variable region, wherein the third heavy chain variable region comprises three heavy chain variable regions CDRs as follows:

H-CDR1 having an amino acid sequence as set forth in SEQ ID NO: 3;
H-CDR2 having an amino acid sequence as set forth in SEQ ID NO: 4; and
H-CDR3 having an amino acid sequence as set forth in SEQ ID NO: 5; and
the third light chain variable region comprises three light chain variable regions CDRs as follows:

L-CDR1 having an amino acid sequence as set forth in SEQ ID NO: 6;

L-CDR2 having an amino acid sequence as set forth in SEQ ID NO: 7; and
L-CDR3 having an amino acid sequence as set forth in SEQ ID NO: 8.

In another preferred embodiment, the MUC17 antigen-binding domain comprises a third heavy chain variable region as set forth in SEQ ID NO: 1 or 9, and a third light chain variable region as set forth in SEQ ID NO: 2 or 10.

In another preferred embodiment, the MUC17 antigen-binding domain comprises a third heavy chain variable region having at least 80%, 90%, 95%, 96%, 97%, 98%, or 99% sequence identity (with CDR sequences unchanged or substantially unchanged) to an amino acid sequence as set forth in SEQ ID NO: 1 or 9; and a third light chain variable region having at least 80%, 90%, 95%, 96%, 97%, 98%, or 99% sequence identity (with CDR sequences unchanged or substantially unchanged) to an amino acid sequence as set forth in SEQ ID NO: 2 or 10.

In another preferred embodiment, the second targeting domain is selected from the group consisting of: a CD3 antigen-binding domain, a CD28 antigen-binding domain, a CD40 antigen-binding domain, a CD137 antigen-binding domain, a CD40L sequence, or a CD137L sequence.

In another preferred embodiment, the third targeting domain is selected from the group consisting of: a CD3 antigen-binding domain, a CD28 antigen-binding domain, a CD40 antigen-binding domain, a CD137 antigen-binding domain, a CD40L sequence, or a CD137L sequence.

In another preferred embodiment, the multispecific T-cell engager further comprises an Fc fragment.

In another preferred embodiment, the Fc fragment is derived from IgG1 or IgG4.

In another preferred embodiment, the Fc fragment is derived from IgG1.

In another preferred embodiment, the Fc fragment derived from IgG1 comprises mutations selected from the group consisting of:

N297A;
L234F/L235E/P331S;
Y349C/K370E/K409D/K439E;
S354C/D356K/E357K/D399K;
S354C/T366W;
S354C/T366W/H435R/Y436F;
Y349C/T366S/L368A/Y407V;
L234F/L235E/P331S/Y349C/K370E/K409D/K439E;
L234F/L235E/P331S/S354C/D356K/E357K/D399K;
L234F/L235E/P331S/S354C/T366W;
L234F/L235E/P331S/Y349C/T366S/L368A/Y407V;
N297A/Y349C/K370E/K409D/K439E;
N297A/S354C/D356K/E357K/D399K;
N297A/S354C/T366W;
N297A/Y349C/T366S/L368A/Y407V; and/or
S267E; S267E/G236D; S267E/S239D; S267E/L328F.

In another preferred embodiment, the Fc fragment derived from IgG1 comprises mutations selected from the group consisting of:

L234A/L235A/G237A;
S354C/T366W;
S354C/T366W/H435R/Y436F;
Y349C/T366S/L368A/Y407V.

In another preferred embodiment, the multispecific T-cell engager is a bispecific/trispecific T-cell engager.

In another preferred embodiment, the multispecific T-cell engager is a bispecific T-cell engager.

In another preferred embodiment, the trispecific T-cell engager comprises:

a first targeting domain, which is a CD28 antigen-binding domain;
a second targeting domain, which is a CD3 antigen-binding domain; and
a third targeting domain, which comprises one or more MUC17 antigen-binding domains.

In another preferred embodiment, the first targeting domain, the second targeting domain, and the third targeting domain each comprise one or more antibody variable regions.

In another preferred embodiment, the multispecific T-cell engager is a trispecific T-cell engager.

In another preferred embodiment, the multispecific T-cell engager is a homodimer or heterodimer.

In another preferred embodiment, the trispecific T-cell engager is a trispecific antibody.

In another preferred embodiment, the trispecific antibody has a symmetric or asymmetric structure.

In another preferred embodiment, the trispecific antibody is a bivalent, trivalent, tetravalent, or multivalent molecule.

In another preferred embodiment, the trispecific antibody is an asymmetric trivalent trispecific antibody.

In another preferred embodiment, the CD3 antigen-binding domain comprises a second heavy chain variable region and a second light chain variable region, wherein the second heavy chain variable region comprises three heavy chain variable regions CDRs as follows:

H-CDR1 having an amino acid sequence as set forth in SEQ ID NO: 17;
H-CDR2 having an amino acid sequence as set forth in SEQ ID NO: 18; and
H-CDR3 having an amino acid sequence as set forth in SEQ ID NO: 19; and
the second light chain variable region comprises three light chain variable regions CDRs as follows:

L-CDR1 having an amino acid sequence as set forth in SEQ ID NO: 20;
L-CDR2 having an amino acid sequence as set forth in SEQ ID NO: 21; and
L-CDR3 having an amino acid sequence as set forth in SEQ ID NO: 22; or
the second heavy chain variable region comprises three heavy chain variable regions CDRs as follows:

H-CDR1 having an amino acid sequence as set forth in SEQ ID NO: 17;
H-CDR2 having an amino acid sequence as set forth in SEQ ID NO: 18; and
H-CDR3 having an amino acid sequence as set forth in SEQ ID NO: 51; and
the second light chain variable region comprises three light chain variable regions CDRs as follows:

L-CDR1 having an amino acid sequence as set forth in SEQ ID NO: 20;
L-CDR2 having an amino acid sequence as set forth in SEQ ID NO: 21; and
L-CDR3 having an amino acid sequence as set forth in SEQ ID NO: 52.

In another preferred embodiment, the CD3 antigen-binding domain comprises a second heavy chain variable region having at least 80%, 90%, 95%, 96%, 97%, 98%, or 99% sequence identity to an amino acid sequence as set forth in SEQ ID NO: 15 or 23, and a second light chain variable region having at least 80%, 90%, 95%, 96%, 97%, 98%, or 99% sequence identity to an amino acid sequence as set forth in SEQ ID NO: 16 or 24.

In another preferred embodiment, the CD3 antigen-binding domain comprises a second heavy chain variable region as set forth in SEQ ID NO:15, and a second light chain variable region as set forth in SEQ ID NO: 16; or a second heavy chain variable region as set forth in SEQ ID NO: 23, and a second light chain variable region as set forth in SEQ ID NO: 24.

In another preferred embodiment, the CD28 antigen-binding domain comprises a first heavy chain variable region and a first light chain variable region, wherein the first heavy chain variable region comprises three heavy chain variable regions CDRs as follows:

H-CDR1 having an amino acid sequence as set forth in SEQ ID NO: 30;
H-CDR2 having an amino acid sequence as set forth in SEQ ID NO: 31 or 53; and
H-CDR3 having an amino acid sequence as set forth in SEQ ID NO: 32; and
the first light chain variable region comprises three light chain variable regions CDRs as follows:

L-CDR1 having an amino acid sequence as set forth in SEQ ID NO: 33;
L-CDR2 having an amino acid sequence as set forth in SEQ ID NO: 34; and
L-CDR3 having an amino acid sequence as set forth in SEQ ID NO: 35.

In another preferred embodiment, the CD28 antigen-binding domain comprises a first heavy chain variable region having at least 80%, 90%, 95%, 96%, 97%, 98%, or 99% sequence identity to an amino acid sequence as set forth in SEQ ID NO: 28 or 50, and a first light chain variable region having at least 80%, 90%, 95%, 96%, 97%, 98%, or 99% sequence identity to an amino acid sequence as set forth in SEQ ID NO: 29.

In another preferred embodiment, the CD28 antigen-binding domain comprises a first heavy chain variable region 28 or 50, and a first light chain variable region as set forth in SEQ ID NO: 29.

In another preferred embodiment, the trispecific antibody is a homodimer or a heterodimer.

In another preferred embodiment, the first targeting domain and the second targeting domain form a hetero-

4

dimeric structure.

In another preferred embodiment, the first targeting domain comprises an anti-CD28 Fv domain, a Fab domain, or an anti-CD28 scFv domain.

In another preferred embodiment, the second targeting domain comprises an anti-CD3 Fv domain, a Fab domain, or an anti-CD3 scFv domain.

In another preferred embodiment, the third targeting domain comprises an anti-MUC17 Fab domain.

In another preferred embodiment, the trispecific antibody comprises the following structure from N-terminus to C-terminus:

(a) a first chain: $VL_1$-L1-$VH_2$-L2-$VL_2$-L3-$VH_1$-L4-Fc1;
(b) a second chain: $VH_3$-CH1-Fc2; and
(c) a third chain: $VL_3$-CL;

or the trispecific antibody comprises the following structure from N-terminus to C-terminus:

(a) a first chain: $VL_1$-L1-$VH_1$-L2-$VL_2$-L3-$VH_2$-L4-Fc1;
(b) a second chain: $VH_3$-CH1-Fc2; and
(c) a third chain: $VL_3$-CL;

wherein,

$VH_1$ is a first heavy chain variable region, and $VL_1$ is a first light chain variable region;
$VH_2$ is a second heavy chain variable region, and $VL_2$ is a second light chain variable region;
$VH_3$ is a third heavy chain variable region, and $VL_3$ is a third light chain variable region;
CL is a light chain constant region, and CH1 is a CH1 domain;
L1, L2, L3 and L4 are each independently none, a bond or a linker;
Fc1 or Fc2 is independently an Fc element; and
"-" represents a peptide bond.

In another preferred embodiment, the linker is a rigid linker or a flexible linker.

In another preferred embodiment, the L1, L2, L3, and L4 are each independently none or (GS)n, (G3S)n, or (G4S) n (with n selected from 1 to 6).

In another preferred embodiment, the trispecific antibody is co-administered with a checkpoint inhibitor.

In another preferred embodiment, the trispecific antibody is co-administered with an anti-PD1 and/or anti-PDL1 antagonist.

In another preferred embodiment, the light chain constant region is a human Kappa light chain constant region.

In another preferred embodiment, the $VH_1$ and $VL_1$ are the first heavy chain variable region and the first light chain variable region, respectively, comprised in the CD28 antigen-binding domain.

In another preferred embodiment, the $VH_2$ and $VL_2$ are the second heavy chain variable region and the second light chain variable region, respectively, comprised in the CD3 antigen-binding domain.

In another preferred embodiment, the $VH_3$ and $VL_3$ are the third heavy chain variable region and the third light chain variable region, respectively, comprised in the MUC17 antigen-binding domain.

In another preferred embodiment, the VH1 comprises: HCDR1, HCDR2, and HCDR3 with amino acid sequences as set forth in SEQ ID NO: 30, SEQ ID NO: 31 and SEQ ID NO: 32, respectively; and the VL1 comprises: LCDR1, LCDR2, and LCDR3 with amino acid sequences as set forth in SEQ ID NO: 33, SEQ ID NO: 34 and SEQ ID NO: 35, respectively; or the VH1 comprises: HCDR1, HCDR2 and HCDR3 with amino acid sequences as set forth in SEQ ID NO: 30, SEQ ID NO: 53 and SEQ ID NO: 32, respectively; and the VL1 comprises: LCDR1, LCDR2, and LCDR3 with amino acid sequences as set forth in SEQ ID NO: 33, SEQ ID NO: 34 and SEQ ID NO: 35, respectively.

In another preferred embodiment, the VH2 comprises: HCDR1, HCDR2 and HCDR3 with amino acid sequences as set forth in SEQ ID NO: 17, SEQ ID NO: 18 and SEQ ID NO: 19, respectively; and the VL2 comprises: LCDR1, LCDR2 and LCDR3 with amino acid sequences as set forth in SEQ ID NO: 20, SEQ ID NO: 21 and SEQ ID NO: 22, respectively; or the VH2 comprises: HCDR1, HCDR2 and HCDR3 with amino acid sequences as set forth in SEQ ID NO: 17, SEQ ID NO: 18 and SEQ ID NO: 51, respectively; and the VL2 comprises: LCDR1, LCDR2 and LCDR3 with amino acid sequences as set forth in SEQ ID NO: 20, SEQ ID NO: 21 and SEQ ID NO: 52, respectively.

In another preferred embodiment, the VH3 comprises: HCDR1, HCDR2 and HCDR3 with amino acid sequences as set forth in SEQ ID NO: 3, SEQ ID NO: 4 and SEQ ID NO: 5; and the VL3 comprises: LCDR1, LCDR2 and LCDR3 with amino acid sequences as set forth in SEQ ID NO: 6, SEQ ID NO: 7 and SEQ ID NO: 8, respectively.

In another preferred embodiment, in the trispecific antibody,

the first chain has an amino acid sequence as set forth in SEQ ID NO: 38 or 42.

In another preferred embodiment, in the trispecific antibody,

the second chain has an amino acid sequence is as set forth in SEQ ID NO: 40; and/or

the third chain has an amino acid sequence as set forth in SEQ ID NO. 14.

In another preferred embodiment, the trispecific antibody further comprises active fragments and/or derivatives thereof, wherein the derivatives of the trispecific antibody have at least 86%, 87%, 88%, 89%, 90%, 91%, 92%, 93%, 94%, 95%, 96%, 97%, 98%, or 99% sequence identity to the trispecific antibody of the present invention.

In a second aspect of the present invention, there is provided a polynucleotide encoding the multispecific T-cell engager according to the first aspect of the present invention.

In a third aspect of the present invention, there is provided a vector comprising the polynucleotide according to the second aspect of the present invention.

In another preferred embodiment, the vector comprises a plasmid, a phage, a yeast plasmid, a plant cell virus, a mammalian cell virus such as an adenovirus, a retrovirus, or other vectors.

In a fourth aspect of the present invention, there is provided a host cell comprising the vector according to the third aspect of the present invention or having a genome incorporating the polynucleotide according to the second aspect of the present invention.

In another preferred embodiment, the host cell is a eukaryotic cell, preferably a mammalian cell.

In a fifth aspect of the present invention, there is provided a method for preparing the multispecific T-cell engager according to the first aspect of the present invention, comprising the steps of:

(i) culturing the host cell according to the fourth aspect of the present invention under suitable conditions to obtain a mixture comprising the multispecific T-cell engager according to the first aspect of the present invention;

(ii) purifying and/or separating the mixture obtained in step (i) to obtain the multispecific T-cell engager according to the first aspect of the present invention.

In a sixth aspect of the present invention, there is provided a pharmaceutical composition, comprising:

(I) the multispecific T-cell engager according to the first aspect of the present invention; and

(II) a pharmaceutically acceptable carrier.

In another preferred embodiment, the pharmaceutical composition further comprises a further pharmaceutically active reagent.

In another preferred embodiment, the further pharmaceutically active reagent comprises one or more checkpoint modulators or chemotherapeutic agents.

In another preferred embodiment, the checkpoint modulators comprise, but are not limited to, anti-PD1 and/or anti-PDL1 antagonists.

In another preferred embodiment, the pharmaceutical composition is an injectable dosage form. In a seventh aspect of the present invention, there is provided an immunoconjugate, comprising:

(a) the multispecific T-cell engager according to the first aspect of the present invention; and

(b) a conjugated moiety selected from the group consisting of: a detectable marker, a drug, a toxin, a cytokine, a radionuclide, an enzyme, or combinations thereof.

In another preferred embodiment, the conjugated moiety is partially selected from: a fluorescent or luminescent marker; a radioactive marker; an MRI (Magnetic Resonance Imaging) or CT (Computerized Tomography) contrast agent; or an enzyme, a radionuclide, a biotoxin, a cytokine (e.g. IL-2, etc.), an antibody, an antibody Fc fragment, an antibody scFv fragment, a gold nanoparticle/nanorod, a virus particle, a liposome, or a magnetic nanoparticle, each of which is capable of producing a detectable product.

In an eighth aspect of the present invention, there is provided use of the multispecific T-cell engager according to the first aspect of the present invention, the pharmaceutical composition according to the sixth aspect of the present invention, or the immunoconjugate according to the seventh aspect of the present invention in preparation of: (a) a detection reagent or kit; and/or (b) a medicament for preventing and/or treating a cancer/tumor.

In another preferred embodiment, the cancer/tumor is MUC17-associated cancer/tumor.

In another preferred embodiment, the cancer/tumor includes solid tumors and hematological tumors.

In another preferred embodiment, the cancer/tumor is a solid tumor.

In another preferred embodiment, the cancer/tumor is selected from the group consisting of: gastric cancer, gastrointestinal cancer, colorectal cancer, esophageal cancer, gastroesophageal cancer, pancreatic cancer, lung cancer, breast cancer, liver cancer, cervical cancer, thyroid cancer, uterine cancer, prostate cancer, or combinations thereof.

In a ninth aspect of the present invention, there is provided a kit, comprising the multispecific T-cell engager according to the first aspect of the present invention, the polynucleotide according to the second aspect of the present invention, the vector according to the third aspect of the present invention, the host cell according to the fourth aspect of the present invention, the pharmaceutical composition according to the sixth aspect of the present invention, or the immunoconjugate according to the seventh aspect of the present invention.

In a tenth aspect of the present invention, there is provided use of the multispecific T-cell engager according to the first aspect of the present invention, the polynucleotide according to the second aspect of the present invention, the vector according to the third aspect of the present invention, the host cell according to the fourth aspect of the present invention, the pharmaceutical composition according to the sixth aspect of the present invention, or the immunoconjugate according to the seventh aspect of the present invention in preparation of a medicament for preventing, treating and/or detecting a cancer/tumor.

In an eleventh aspect of the present invention, there is provided a method for preventing, treating and/or detecting a cancer/tumor, comprising: administering to a subject in need thereof a safe and effective amount of the multispecific T-cell engager according to the first aspect of the present invention, a pharmaceutical composition thereof, or an immunoconjugate thereof.

It should be understood that each of the above technical features of the present invention and each of the technical features specifically described below (e.g., in the Examples) can be combined with each other within the scope of the present invention to thus form new or preferred technical solutions, which will not be repeated here one by one due to space limitation.

## BRIEF DESCRIPTION OF DRAWINGS

FIG. 1 shows the schematic structural diagrams of trispecific antibodies of the present invention, where FIG. 1A shows the schematic structural diagram of a CD28-44/SP34-Dia+44H4-KIH antibody, and FIG. 1B shows the schematic structural diagram of a CD28-44/SP34-ScFv+44H4-KIH antibody;

FIG. 2 shows the ELISA analysis of the antigen binding capacity of an anti-MUC17*CD3*CD28 trispecific antibody to MUC17 (FIG. 2A), CD3 (FIG. 2B), and CD28 (FIG. 2C);

FIG. 3 shows an assay on the binding capacity of the anti-MUC17*CD3*CD28 trispecific antibody to MUC17 on the surface of human colon adenocarcinoma cells LS174T (FIG. 3A), as well as to CD3/CD28 on the surface of human T cells (FIG. 3B) using the flow cytometer;

FIG. 4 shows the cytotoxicity of the anti-MUC17*CD3*CD28 trispecific antibody against target cells LS174T-Luciferase, with FIGs. 4A and 4B illustrating the results of two independent repeated experiments, in which effector cells used are sourced from different human individuals;

FIG. 5 shows the superiority evaluation of the anti-MUC17*CD3*CD28 trispecific antibody versus bispecific antibodies, where a mutant SP34-Hu-IgG1 (A101D) in FIG. 5A no longer binds to CD3-Epsilon, and a mutant Anti-CD28-Hu-IgG1 (H35A+H96A) in FIG. 5B no longer binds to CD28;

FIG. 6 shows the activity of the trispecific antibody of the present invention and its mutants in killing target cells (FIG. 6A) and in stimulating T cell secretion of IFN-gamma (FIG. 6B);

FIG. 7 shows the superiority of the anti-MUC17*CD3*CD28 trispecific antibody over CODV-IgG, where FIG. 7A shows a comparison of the cytotoxicity against target cells, and

FIG. 7B shows a comparison of the ability to stimulate CD3-positive T cell secretion of IFN-gamma;

FIG. 8 shows the in vitro safety evaluation of the anti-MUC17*CD3*CD28 trispecific antibody, where an SP34-Hu-IgG1 monoclonal antibody is shown to strongly stimulate PBMC secretion of IL-2 (FIG. 8A) and IFN-gamma (FIG. 8B), as well as PBMC proliferation (FIG. 8C);

FIG. 9 shows the SEC purity analysis of the anti-MUC17*CD3*CD28 trispecific antibody, where FIG. 9A and FIG. 9B show the HPLC-SEC spectra of CD28-44/SP34-Dia+44H4-KIH and CD28-44/SP34-ScFv+44H4-KIH, respectively;

FIG. 10 shows the CE-SDS analysis of the anti-MUC17*CD3*CD28 trispecific antibody, where FIGs. 10A and 10B show the NR-CE-SDS and R-CE-SDS spectra of CD28-44/SP34-Dia+44H4-KIH, respectively, and FIGs. 10C and 10D show the NR-CE-SDS and R-CE-SDS spectra of CD28-44/SP34-ScFv+44H4-KIH, respectively; and

FIG. 11 shows the animal tumor suppression efficacy (including the body weight and tumor volume of each mouse) of the anti-MUC17*CD3*CD28 trispecific antibody.

## DETAILED DESCRIPTION OF THE PREFERRED EMBODIMENTS

After extensive and in-depth studies, the inventors have unexpectedly developed for the first time an anti-MUC17 multispecific T-cell engager. The multispecific T-cell engager of the present invention preferably comprises humanized domains targeting MUC17, CD3, and CD28. The multispecific T-cell engager of the present invention, in addition to binding CD3, can also target CD28, allowing for providing costimulatory signals to T cells to induce stronger and more effective cytotoxicity. The trispecific antibody can effectively overcome the drug resistance of solid tumors to T-cell engagers. The present invention has been completed on this basis.

## Terms

Unless otherwise defined, all technical and scientific terms used herein have the same meaning as commonly understood by those of ordinary skill in the art to which the present invention belongs.

The term "about" may refer to a value or set that is within an acceptable margin of error for a particular value or set as determined by those of ordinary skill in the art, which will depend in part on how the value or set is measured or determined.

As used herein, the terms "contain" or "comprise (comprising)" may be open-ended, semi-closed and closed-ended. In other words, these terms also include "consisting essentially of", or "consisting of".

## MUC17

MUC17 is a high-molecular-weight O-glycosylated mucin that belongs to the SEA (Sea urchin sperm protein, Enterokinase, and Agrin) family of transmembrane mucins, along with several other mucins (such as MUC1, 3, 12, 13, and 16). The transmembrane mucins are crucial for maintaining the mucosal barrier function, and they can prevent pathogen invasion at mucosal surfaces, and are also associated with the development of inflammatory bowel diseases. Through analysis of HPA (Human Protein Atlas, a genomics database) data, the applicants found that MUC17 RNAs were primarily expressed in colorectal and gastric-related organs (small intestine, duodenum, rectum, colon and stomach). Data from clinical cancer studies indicate that MUC17 is highly expressed in a variety of gastrointestinal tumors, including gastric and colorectal cancers, making MUC17 a potential target related to gastrointestinal tumors.

## T-cell Engagers

Bispecific T-cell engagers (BiTEs) are bispecific antibodies formed by the fusion of fragments from two distinct antibodies. The BiTEs can bind CD3 molecules on the surface of T cells as well as antigenic molecules on the surface of tumor cells, respectively, thereby inducing the killing of tumor cells by T cells, and this process is not restricted by MHC.

In February 2022, Sanofi's R&D team published their research progress on HER2 × CD3 × CD28-targeting trispecific T-cell engagers (TriTEs) (with a CODV-IgG structure) in Nature (Reference: Seung E, Xing Z, Wu L, et al. A trispecific antibody targeting HER2 and T cells inhibits breast cancer growth via CD4 cells[J]. Nature, 2022, 603(7900): 328-334.). The study demonstrated that these TriTEs could more effectively stimulate IL-2 secretion and activate the NF-κB signaling pathway compared to BiTEs, while exhibiting stronger cytotoxic activity. In immunodeficient NSG mice reconstituted with primary human CD3-positive T cells, the TriTEs increased granzyme expression in CD8-positive T cells by 6.8-fold, and HER2×CD3×CD28 could still induce tumor regression at doses as low as 10μg/kg. The HER2×CD3×CD28-targeting TriTEs described in this paper have entered Phase 1 clinical development and show potential for treating HER2-positive solid tumors.

## Antibody

Typically, an "antibody" is also referred to as an "immunoglobulin" that may be a natural or conventional antibody, in which two heavy chains are linked to each other by a disulfide bond and each heavy chain is linked to a light chain by a disulfide bond. There are two types of light chains, namely, $\lambda$(l) and $\kappa$(k). There are five main heavy chain types (or isotypes), which determine the functional activity of antibody molecules: IgM, IgD, IgG, IgA, and IgE. Each type of chain comprises a different sequence of domains. The light chain comprises two domains or regions, namely, a variable domain (VL) and a constant domain (CL). The heavy chain comprises four domains, namely, a heavy chain variable region (VH) and three constant regions (CH1, CH2 and CH3, collectively referred to as CH). The variable regions of the light chain (VL) and heavy chain (VH) both determine binding recognition and specificity for antigens. The light chain constant domain (CL) and the heavy chain constant regions (CHs) give important biological properties, such as antibody chain binding, secretion, transplacental mobility, complement binding and binding to an Fc receptor (FcR). An Fv fragment is an N-terminus moiety of an immunoglobulin Fab fragment and consists of one light chain and the variable moieties of one heavy chain. The specificity of an antibody depends on the structural complementarity between an antibody binding site and an antigen determining interval. The antibody binding site consists of residues originating primarily from highly variable regions or complementary determining regions (CDRs). Occasionally, residues originating from non-highly variable or framework regions (FR) affect the overall domain structure and thus the binding site. The complementary determining regions or CDRs refer to amino acid sequences that collectively define the binding affinity and the specificity of the natural Fv region of the natural immunoglobulin binding site. The light and heavy chains of the immunoglobulin each have three CDRs, which are also referred to as CDR1-L, CDR2-L, CDR3-L and CDR1-H, CDR2-H, CDR3-H. The conventional antibody-antigen binding site thus comprises six CDRs, including a collection of CDRs from each v region of the heavy and light chains.

Within the amino acid sequence of a given antibody's light chain variable region or heavy chain variable region, the exact amino acid sequence boundary of each CDR can be determined using any one or a combination of many well-known antibody CDR assignment systems, including, for example: Chothia, Kabat, AbM, Contact, the international Immuno GeneTics database (IMGT), the EU numbering system, and the like.

It should be understood that the exact amino acid sequence boundaries of the CDRs in the present invention may be optionally defined using the different assignment systems as mentioned above. Preferably, unless otherwise indicated, the positions of residues (including residues in both heavy and light chain variable regions) in the variable region of an antibody, when mentioned in the present invention, refer to positions numbered according to the Kabat numbering system.

As used herein, the term "variable" denotes that some moieties of the variable region of an antibody are somewhat different in sequence, which contribute to the binding and specificity of various particular antibodies to their particular antigens. However, variability is not uniformly distributed across the variable region of an antibody. It is concentrated in three fragments in the so-called complementary determining regions (CDRs) or hypervariable regions in the light and heavy chain variable regions. A conserved moiety in the variable region is called the framework region (FR). The variable regions of the natural heavy and light chains each comprise four FRs, which are roughly in a β-sheet configuration and linked by three CDRs that form a linking loop, and which in some cases may form a partially β-sheet structure. The CDRs in each chain are close together through the FRs and form, together with the CDRs of the other chain, the antigen-binding site of the antibody (see Kabat et al., NIH Publ. No. 91-3242, Vol. I, pp. 647-669 (1991)). The constant regions are not directly involved in antibody-antigen binding, but exhibit different effector functions. For example, they are involved the antibody-dependent cytotoxicity of the antibody.

As used herein, the term "framework region" (FR) refers to an amino acid sequence inserted between CDRs, that is, those moieties of the light and heavy chain variable regions of relatively conserved immunoglobulins among different immunoglobulins in a single species. The light and heavy chains of an immunoglobulin each have four FRs, referred to as FR1-L, FR2-L, FR3-L, FR4-L and FR1-H, FR2-H, FR3-H, FR4-H, respectively. Accordingly, the light chain variable domain may thus be referred to as (FR1-L)-(CDR1-L)-(FR2-L)-(CDR2-L)-(FR3-L)-(CDR3-L)-(FR4-L), and the heavy chain variable domain may thus be represented as (FR1-H)-(CDR1-H)-(FR2-H)-(CDR2-H)-(FR3-H)-(CDR3-H)-(FR4-H). Preferably, the FR in the present invention is a human antibody FR or a derivative thereof, and the derivative of the human antibody FR is substantially identical to the FR of a naturally occurring human antibody, i.e., with the sequence identity being up to 85%, 90%, 95%, 96%, 97%, 98% or 99%.

Once the amino acid sequence of the CDR is known, those skilled in the art can readily determine the framework regions FR1-L, FR2-L, FR3- L, FR4-L and/or FR1-H, FR2-H, FR3-H, FR4-H.

As used herein, the term "human framework region" is a framework region that is substantially identical (about 85% or more, specifically 90%, 95%, 97%, 99%, or 100%) to the framework region of a naturally occurring human antibody.

As used herein, the terms "fragment", "derivative" and "analog" each refer to a polypeptide that maintains substantially the same biological function or activity as the antibody of the present invention. The polypeptide fragment, derivative or analog of the present invention may be: (i) a polypeptide having one or more conserved or non-conserved amino acid residues (preferably conserved amino acid residues) substituted in such a way that the substituted amino acid residues may or may not be encoded by the genetic code, or (ii) a polypeptide having substituent groups in one or more amino acid residues, or (iii) a polypeptide formed by fusion of a mature polypeptide with a further compound (e.g., a compound for extending the half-life period of the polypeptide, for example, polyethylene glycol); or (iv) a polypeptide formed by fusion of an additional amino acid sequence to the current polypeptide sequence (e.g., a leading sequence or a secretory sequence or a sequence for purifying the current polypeptide or a proteinogen sequence, or a fusion protein formed with a 6His tag). According to the teachings herein, these fragments, derivatives and analogs shall fall within the scope of what is known to those skilled in the art.

As used herein, the terms "antibody fragment", "antigen-binding fragment", "targeting domain", or "antigen-binding domain" refer to a portion of an antibody, such as F(ab')2, F(ab)2, Fab', Fab, Fv, single-chain Fvs (scFv), single-chain antibodies, disulfide-linked Fvs (sdFv), fragments containing VL or VH domains, fragments produced by Fab expression libraries, and anti-idiotypic (anti-Id) antibodies. Regardless of structure, the antibody fragments bind to the same antigen recognized by the intact antibody. Examples of the targeting domains of the present invention include, but are not limited to, Fab, Fab', F(ab')2, Fv fragments, single-chain Fv (scFv) fragments, and single-domain fragments.

EP 4 682 172 A1

The "Fv" fragment is the smallest fragment of the antibody comprising the complete target recognition and binding site. This region consists of a dimer (VH-VL dimer) of variable domains of one heavy chain and one light chain that are bound in a tight non-covalent manner. In this configuration, three CDRs in each variable domain interact to define the target binding site on the surface of the VH-VL dimer. Typically, six CDRs endow the antibody with the target binding specificity. In some cases, however, even a single variable domain (or only half of an Fv comprising only three CDRs with target specificity) may be able to recognize and bind a target, despite its lower affinity than the entire binding site.

The antibody-binding fragment "single-chain Fv" or "scFv" comprises the VH and VL domains of the antibody, with these domains present in a single polypeptide chain. Generally, the Fv polypeptide further comprises a polypeptide linker between the VH and VL domains, which enables the scFv to form a structure facilitating target binding.

The "single-domain fragment" consists of a single VH or VL domain that shows sufficient affinity to the coronavirus RBD. In a specific embodiment, the single-domain fragment is camelidized.

As used herein, the term "light chain constant region (CL)" comprises an amino acid sequence CL derived from an antibody light chain. Preferably, the light chain constant region comprises at least one of a constant κ domain or a constant λ domain.

As used herein, the term "heavy chain constant region (CH)" refers to an amino acid sequence derived from an immunoglobulin heavy chain. A polypeptide comprising a heavy chain constant region comprises at least one of: a CH1 domain, a hinge region (e.g., upper, middle, and/or lower hinge region) domain, a CH2 domain, a CH3 domain, or a variant or fragment thereof. It should be understood that the heavy chain constant regions may be modified such that their amino acid sequences differ from those of naturally occurring immunoglobulin molecules.

As used herein, the term "antigen" or "target antigen" refers to a molecule, or a moiety thereof, capable of being bound by an antibody or antibody-like binding protein. This term further refers to a molecule, or a moiety thereof, that can be used in an animal to produce an antibody capable of binding to an epitope of the antigen. The target antigen may have one or more epitopes. For each target antigen recognized by an antibody or an antibody-like binding protein, the antibody-like binding protein is capable of competing with an intact antibody that recognizes the target antigen.

As used herein, the term "linker" refers to one or more amino acid residues that are inserted into the immunoglobulin structural domains to provide sufficient mobility to the light and heavy chain domains for pleating of the domains into an exchanged dual variable region immunoglobulin. Examples of appropriate linkers include single glycine (Gly) or serine (Ser) residues, and the identifications and sequences of amino acid residues in the linker can vary depending on the type of secondary structure elements to be achieved in the linker. The preferred linker may be (GS)n, (G3S)n, or (G4S)n (with n selected from 1 to 6).

As used herein, a "variant" of an antibody, antibody fragment, or antibody domain refers to an antibody, antibody fragment, or antibody domain that: (1) has at least 80%, 85%, 90%, 95%, 96%, 97%, 98%, or 99% sequence identity to the original antibody, antibody fragment, or antibody domain, and (2) specifically binds to the same target as the original antibody, antibody fragment, or antibody domain. It should be understood that when sequence identity is expressed in the form of "at least x% identical" or "at least x% identity", such embodiments include any and all numerical percentages equal to or above the lower limit. Furthermore, it should be understood that in a case where an amino acid sequence is present in the present application, it shall be interpreted as additionally disclosing or comprising an amino acid sequence having at least 80%, at least 85%, at least 90%, at least 95%, at least 96%, at least 97%, at least 98%, or at least 99% identity thereto.

**T-cell Engagers of the Present Invention**

As used herein, the term "multispecific T-cell engager" refers to a molecule comprising at least two targeting domains with different binding specificities, with at least one targeting domain specifically binding to a T cell surface antigen. In some embodiments, a multispecific inhibitor is a polypeptide comprising a scaffold and two or more immunoglobulin antigen-binding domains that target different antigens or epitopes. In some embodiments, the multispecific T-cell engager is a bispecific antibody or a trispecific antibody.

As used herein, the terms "bispecific antibody" and "bispecific T-cell engager" are used interchangeably herein and refer to antibodies capable of specifically binding to two different antigens (or epitopes).

As used herein, the terms "the trispecific antibody of the present invention", "the trispecific T-cell engager of the present invention" and "3Sbody" are used interchangeably herein and refer to molecules comprising three targeting domains with three different binding specificities. Each targeting domain is capable of specifically binding to a target molecule and inhibiting the biological function of the target molecule upon binding. In some embodiments, a trispecific antagonist is a polymeric molecule comprising two or more peptides. In some embodiments, the targeting domain comprises an antigen-binding domain or CDR of an antibody. In some embodiments, the targeting domain comprises a ligand, or a fragment thereof, that specifically binds to a target protein.

It should be understood that the trispecific T-cell engager of the present invention is essentially a trispecific antibody, which is an antibody molecule capable of specifically binding to three antigens at the same time. Based on symmetry, the trispecific antibodies can be classified into structurally symmetric and asymmetric molecules. Based on the number of binding sites, the bispecific antibodies can be classified into bivalent, trivalent, tetravalent, and multivalent molecules. The two types of trispecific antibodies of the present invention are structurally asymmetric trivalent trispecific antibodies, which are monovalent for each specific target.

As used herein, the term "Fc fragment" or "Fc" refers to an antibody moiety that lacks antigen-binding activity but is initially observed to readily crystallize, and is thus named the Fc fragment (in terms of fragment crystallizability). This fragment corresponds to the paired CH2 and CH3 domains and constitutes the moiety of the antibody molecule that interacts with effector molecules and cells. The Fc fragment described herein can be derived from IgG1, IgG2, and IgG4 antibodies. For specific uses, particular IgG subclasses may be preferable selected. Additionally, the effector function of the antibody may be increased or decreased by introducing one or more mutations into the Fc. Preferably, the Fc fragment may comprise L234A/L235A/G237A mutations to attenuate the interaction between Fc and Fcγ receptors. Furthermore, mutations may be introduced to form a knob-into-hole (KIH) structure. Its primary function is to promote the heterodimerization of two different heavy chains of a bispecific antibody. The structural features are as follows: two different heavy chains form the bispecific antibody, where the CH3 region of one heavy chain is mutated to form a protruding "knob" structure, and the CH3 region of the other heavy chain is mutated to form a recessed "hole" structure, and the knob-into-hole structure is designed to facilitate the correct assembling of the two heterologous antibody heavy chains. Specifically, T at position 366 in the CH3 domain of the first heavy chain is mutated to W with a larger side chain volume to form a protruding "knob" structure; meanwhile, T at position 366 in the CH3 domain of the second heavy chain is mutated to S, L at position 368 is mutated to A, and Y at position 407 is mutated to V, and these three mutations all result in smaller side chain volumes, thereby forming a recessed "hole" structure. Furthermore, S at position 354 of the first heavy chain is mutated to C, and Y at position 349 of the second heavy chain is mutated to C. In this way, an additional covalent disulfide bond is introduced between the first and second heavy chains after the formation of the knob-into-hole structure, thereby further stabilizing the resulting knob-into-hole structure.

Specifically, for the knob-into-hole form, the T366W mutation is introduced in the first Fc to create the "knob", and mutations T366S, L368A, and Y407V can be introduced in the second Fc to form the "hole". For the charge pair form, ionic interactions are stabilized by introducing linkage charged residues in opposing Fc domains, thereby facilitating heterodimerization, for example, D356K, E357K, and D399K in the first Fc domain, and K370E, K409D, and K439E mutations in the second Fc domain, or combinations thereof (residues are numbered according to the Kabat EU numbering system). In addition, cysteine can be introduced to stabilize the pairing of heterodimers, for example, S234C in the first Fc and Y349C in the second Fc, or Y349C in the first Fc and S344C in the second Fc. H at position 435 of the "hole" chain is mutated to R and Y at position 436 to F, in such a way that the binding effect of the "hole" chain to Protein A can be eliminated, so as to remove the homodimer of the "hole" chain in the purification step.

The multispecific molecules of the present invention may include various structures selected from the group consisting of: asymmetric IgG-like antibodies (e.g., triomabs/quadromas); knobs-into-holes antibodies; Cross MAbs; electrostatically matched antibodies; LUZ-Y; strand-exchange engineered domain (SEED) bodies; Fab-exchange antibodies; symmetric IgG-like antibodies; two-in-one antibodies; cross-linked monoclonal antibodies, mAb2; Cov X-body; dual variable domain (DVD)-Ig fusion proteins; IgG-like bispecific antibodies; Ts2Ab; BsAb; scFv/Fc fusions; dual (scFv)2-Fabs; F(ab)2 fusion proteins; dual-acting or Bis-Fab; Dock-and-Lock (DNL); Fab-Fv; scFv antibodies and diabodies (e.g., BiTEs); tandem diabodies (Tandabs); DARTs; single-chain diabodies; TCR-like antibodies; and human serum albumin scFv fusion proteins, COMBODIES, and IgG/non-IgG fusion proteins.

**Encoding Nucleic Acid and Expression Vector**

The present invention further provides a polynucleotide molecule encoding the aforementioned antibody or a fragment thereof. The polynucleotide of the present invention may be in a DNA or RNA form. The DNA form includes cDNA, genomic DNA or synthetic DNA. The DNA may be single-stranded or double-stranded. The DNA may be a coding or non-coding strand. A coding region sequence encoding a mature polypeptide may be identical to a coding region sequence of the antibody of the present invention or a degenerate variant thereof. As used herein, the "degenerate variant" in the present invention refers to a nucleic acid sequence that encodes a polypeptide having the same amino acid sequence as the polypeptide of the present invention, but differs in its coding region sequence.

The polynucleotide encoding a mature polypeptide of the present invention includes: a coding sequence encoding only the mature polypeptide; a coding sequence of the mature polypeptide and various additional coding sequence; a coding sequence (and optionally additional coding sequences) and a non-coding sequence of the mature polypeptide.

The term "polynucleotide encoding a polypeptide" may be a polynucleotide comprising the encoded polypeptide, or a polynucleotide comprising an additional coding and/or non-coding sequence(s).

The present invention further relates to a polynucleotide that hybridizes with the sequences described above and has at least 50%, preferably at least 70%, or more preferably at least 80% identity between the two sequences. The present invention relates in particular to a polynucleotide that is hybridizable with the polynucleotide described in the present invention under stringent conditions. In the present invention, the "stringent conditions" refer to: (1) hybridization and elution under conditions of lower ionic strength and higher temperature, such as $0.2 \times$ SSC, 0.1% SDS at 60°C; or (2) hybridization with a denaturant added, such as 50% (v/v) formamide, 0.1% bovine serum/0.1% Ficoll, at 42°C, etc.; or (3) hybridization occurring only when the identity between two sequences is at least more than 90%, preferably more than 95%. Moreover, the polypeptide encoded by the hybridizable polynucleotide has the same biological function and activity as the mature polypeptides as set forth in SEQ ID NO. 4 and SEQ ID NO. 9.

The full-length nucleotide sequence of the antibody of the present invention or fragments thereof can typically be obtained by PCR amplification, recombination or synthetic methods. A possible method is to synthesize a related sequence by the synthetic method, especially when the fragment length is short. Typically, a fragment with a very long sequence can be obtained by first synthesizing a plurality of small fragments and then ligating them. In addition, the heavy chain coding sequences and expression tags (e.g., 6His) can be fused together to form a fusion protein.

Once the related sequence has been obtained, the recombination method may be used to obtain the sequence on a large scale. This is typically done by cloning into a vector, transferring into a cell, and then isolating the related sequence from proliferated host cells by a conventional method. The biomolecules (nucleic acids, proteins, etc.) involved in the present invention comprise biomolecules in isolated form.

At present, it is already possible to obtain a DNA sequence encoding a protein of the present invention (or a fragment thereof, or a derivative thereof) exclusively by chemical synthesis. Then, this DNA sequence can be introduced into various existing DNA molecules (or, for example, vectors) and cells known in the art. In addition, mutations may be introduced into the protein sequence of the present invention by chemical synthesis.

The present invention further relates to vectors comprising appropriate DNA sequences as described above, as well as appropriate promoters or control sequences. These vectors can be used to transform appropriate host cells to enable them to express the protein.

The host cells may be prokaryotic cells, such as bacterial cells; or lower eukaryotic cells, such as yeast cells; or higher eukaryotic cells, such as mammalian cells. Representative examples include: bacterial cells of Escherichia coli, Streptomyces spp. and Salmonella typhimurium; fungal cells such as yeast; insect cells of Drosophila S2 or Sf9; and animal cells such as CHO, COS7 and 293 cells, etc.

Transformation of the host cells with recombinant DNAs can be performed by conventional techniques known to those skilled in the art. When the host is a prokaryote such as Escherichia coli, competent cells capable of absorbing DNA can be harvested after an exponential growth period and then treated with the $CaCl_2$ method, with the steps well known in the art. Another method includes the use of $MgCl_2$. The transformation may also be performed by electroporation, if desired. When the host is an eucaryon, the following DNA transfection methods may be used: calcium phosphate co-precipitation, conventional mechanical methods such as microinjection, electroporation, liposome packaging, etc.

The obtained transformants may be cultured by conventional methods to express the polypeptide encoded by the genes of the present invention. Depending on the host cells used, a medium used during culturing may be selected from a variety of conventional media. The culture is carried out under conditions suitable for the growth of the host cells. After the host cells have grown to an appropriate cell density, a selected promoter is induced with an appropriate method (e.g., temperature shift or chemical induction), and the cells are cultured for an additional period of time.

The recombinant polypeptides in the methods above may be expressed inside the cells or on the cell membranes, or secreted outside the cells. The recombinant proteins may be isolated and purified by various separation methods using their physical, chemical and other properties, if desired. These methods are well known to those skilled in the art. Examples of these methods include but are not limited to: conventional renaturation, treatment with a protein precipitant (a salting-out method), centrifugation, osmotic lysis, ultra-treatment, ultra-centrifugation, molecular sieve chromatography (gel filtration), adsorption chromatography, ion exchange chromatography, high-performance liquid chromatography (HPLC), and various other liquid chromatography techniques, and combinations of these methods.

The antibody of the present invention may be used alone or in combination, binding or conjugation with detectable markers (for a diagnostic purpose), therapeutic agents, PK (protein kinase) modification moieties, or any combination thereof.

The detectable marker for diagnostic purposes includes, but is not limited to: a fluorescent or luminescent marker, a radioactive marker, an MRI (magnetic resonance imaging) or CT (computed tomography) contrast agent, or an enzyme capable of producing a detectable product.

The conjugatable therapeutic agent includes, but is not limited to: an insulin, IL-2, an interferon, a calcitonin, a GHRH peptide, an enteropeptide analog, albumin, an antibody fragment, a cytokine, and a hormone.

**Composition**

The present invention further provides a composition. In a preferred embodiment, the composition is a pharmaceutical composition comprising the aforementioned antibody or an active fragment thereof or a fusion protein thereof, together with a pharmaceutically acceptable carrier. Typically, these substances may be formulated in a non-toxic, inert, and pharmaceutically acceptable aqueous carrier medium, with the pH typically of about 5-8, and preferably about 6-8, although the pH may vary depending on the nature of the substances to be formulated and the condition to be treated. The prepared pharmaceutical composition may be administered via conventional routes, including (but not limited to): oral, respiratory, intratumoral, intraperitoneal, intravenous, or topical administration.

The pharmaceutical composition of the present invention may be used for treating cancer/tumors, in particular solid tumors, especially solid tumors with high MUC17 expression. The pharmaceutical composition of the present invention comprises a safe and effective amount (e.g., 0.001-99 wt%, preferably 0.01-90 wt%, more preferably 0.1-80 wt%) of the aforementioned monoclonal antibody (or a conjugate thereof) of the present invention, and a pharmaceutically acceptable carrier or excipient. Such carriers include (but are not limited to): saline, buffer solutions, glucose, water, glycerol, ethanol, and their combination. The pharmaceutical preparation should be compatible with the route of administration. The pharmaceutical composition of the present invention may be prepared into an injectable form, for example, by conventional methods using normal saline or aqueous solutions containing glucose and other excipients. The pharmaceutical composition in a form such as an injection or a solution should be manufactured under sterile conditions. The dosage of an active ingredient is a therapeutically effective amount, for example, about 1 μg/kg body weight to about 10 μg/kg body weight per day. In addition, the pharmaceutical composition of the present invention may also be used in combination with other therapeutic agents.

When the pharmaceutical composition is used, a safe and effective amount of the immunoconjugate is administered to a mammal, with the safe and effective amount typically of at least about 10 micrograms per kilogram of body weight, and in most cases not more than about 8 milligrams per kilogram of body weight, preferably about 10 micrograms to about 1 milligrams per kilogram of body weight. Without doubt, the specific dose should also take factors such as the route of administration and the patient's health status into consideration, all of which fall within the area of expertise of skilled physicians.

**Application**

The present invention relates to a method for preventing, treating, and/or detecting a cancer/tumor or an autoimmune disease. The method comprises administering to a subject in need thereof an effective amount of the multispecific T-cell engager or trispecific antibody of the present invention. In another aspect, the method for preventing, treating, and/or detecting a cancer/tumor comprises administering to a subject in need thereof an effective amount of one or more expression vectors expressing the trispecific antibody of the present invention. In another preferred embodiment, the cancer/tumor is MUC17-associated cancer/tumor, characterized by a disease/condition in which the number/proportion/activity of MUC17-expressing cells is increased compared to the number/proportion/activity of MUC17-expressing cells in the absence of the disease/condition. The cancer/tumor includes solid tumors and hematological tumors. Preferably, it is selected from the group consisting of: gastric cancer, gastrointestinal cancer, colorectal cancer, esophageal cancer, gastroesophageal cancer, pancreatic cancer, lung cancer, breast cancer, liver cancer, cervical cancer, thyroid cancer, uterine cancer, prostate cancer, or combinations thereof.

**Main Advantages of the Present Invention**

(1) The trispecific antibody of the present invention is capable of targeting a tumor-specific antigen MUC17 and also targeting T cell-expressed CD3 and costimulatory molecules CD28, allowing for stimulating stronger and more effective killin activity against target cells. Moreover, the trispecific antibody shows favorable in vitro safety.

(2) The trispecific antibody of the present invention exhibits high purity and favorable physicochemical properties, avoiding issues such as aggregates, degradation fragments, and incomplete assembly that tend to occur during antibody production and storage, thereby facilitating industrial-scale manufacturing.

The present invention is further described below in conjunction with specific examples. It should be understood that these examples are intended only to illustrate the present invention, rather than limiting the scope of the present invention. In the following examples, experimental methods without specified detailed conditions are generally carried out under conventional conditions as described in Sambrook et al., Molecular Cloning: A Laboratory Manual (New York: Cold Spring Harbor Laboratory Press, 1989), or as recommended by the manufacturers. Unless otherwise stated, percentages and parts are calculated by weight.

**Example 1. Sources and Sequences of Anti-MUC17 Monoclonal Antibody**

According to CN202111500224.5, the amino acid sequence (SEQ ID NO: 1) of the heavy chain variable region of the anti-MUC17 murine monoclonal antibody 44H4 is as follows:

EVQLQQSGAELVKPGASVKLSFTASGFNIKDTYIHWVKQRPEQGLEWIGRIDPANGYT
KYGPRFQGKATITADTASNAAYLQLSSLTSEDTAVYYCARNYGTSYPNAMDYWGQGT
SVTVSS

The amino acid sequence (SEQ ID NO: 2) of the light chain variable region of the anti-MUC17 murine monoclonal antibody 44H4 is as follows:

DIVLTQSPASLAVSLGQRATISCRASESVETYGNSFMHWYQQKPGQPPKLLIYRASSLES
GIPARFSGSGSRTDFTLTITPVEADDVATYFCQQSNEDPYTFGGGTKLEIK

The amino acid sequences of the heavy and light chain variable regions of the murine monoclonal antibody 44H4 were analyzed; and the complementarity determining regions (CDRs) and framework regions of the heavy and light chain variable regions were determined according to the Kabat numbering scheme, respectively. The amino acid sequences of the CDRs in the 44H4 heavy chain variable region are respectively as follows:

H-CDR1: DTYIH (SEQ ID NO: 3), H-CDR2: RIDPANGYTKYGPRFQG (SEQ ID NO: 4), and H-CDR3: NYGTSYPNAMDY (SEQ ID NO: 5), and
the amino acid sequences of the CDRs in the light chain variable region are respectively as follows: L-CDR1: RASESVETYGNSFMH (SEQ ID NO: 6), L-CDR2: RASSLES (SEQ ID NO: 7), and L-CDR3: QQSNEDPYT (SEQ ID NO: 8).

The humanization process of the murine 44H4 is described as follows. The heavy chain variable region of the murine monoclonal antibody 44H4 was aligned with the germline sequence of the heavy chain variable region of a human antibody for homology; IGHV1-46*01 was selected as a humanization template; three heavy chain CDRs of the murine 44H4 were grafted into IGHV1-46*01 to replace the CDRs of IGHV1-46*01 at corresponding positions, respectively; and a fourth framework region was added after H-CDR3 to obtain the amino acid sequence of a CDR-grafted heavy chain variable region. Similarly, the light chain variable region of murine 44H4 was compared with human antibody light chain variable region germline sequences for homology analysis. IGKV7-3*01 was selected as the humanization template. The three light chain CDRs of murine 44H4 were grafted into the framework regions of IGKV7-3*01, replacing the corresponding CDRs of IGKV7-3*01 respectively, and the fourth framework region was added after L-CDR3 to obtain the CDR-grafted light chain variable region amino acid sequence. Based on the CDR-grafted variable regions, certain framework residues were back-mutated (back-mutation refers to substituting specific amino acid residues in the human framework regions with corresponding murine residues at identical positions, where such residues are typically critical for maintaining antibody structure and/or affinity). During the reverse mutation, the amino acid sequence was coded with Kabat, and the position of the site was indicated by the Kabat code.

Preferably, for the CDR-grafted heavy chain variable region, Y at position 27 was reversely mutated to the murine F, T at position 28 was mutated to N, F at position 29 was mutated to I, T at position 30 was mutated to K, M at position 69 was mutated to I, and R at position 71 was mutated to A. For the CDR-grafted light chain variable region, N at position 81 was mutated to D to eliminate the N-glycosylation site of the light chain. The aforementioned heavy and light chain variable regions with mutations were defined as the 44H4 humanized heavy chain variable region (SEQ ID NO: 9) and light chain variable region (SEQ ID NO: 10), respectively. DNAs encoding the aforementioned humanized heavy and light chain variable regions were synthesized by Sangon Biotech (Shanghai) Co., Ltd. Using genetic engineering methods, the synthesized humanized heavy chain variable region was fused with the human IgG1 heavy chain constant region (SEQ ID NO: 11) to obtain the full-length humanized heavy chain, designated as 44H4-Hu-HC (SEQ ID NO: 12). Similarly, the humanized light chain variable region was fused with the human kappa light chain constant region (SEQ ID NO: 13) to obtain the full-length humanized light chain, designated as 44H4-Hu-LC (SEQ ID NO: 14).

The coding genes of 44H4-Hu-HC and 44H4-Hu-LC were cloned into pcDNA3.4 expression vectors, respectively, and the heavy and light chain expression vectors were co-transfected into HEK293F cells using PEI for antibody expression. After 5 days, a FreeStyle™ 293-F cell culture supernatant was collected, and the antibody was purified by Protein A affinity chromatography, with the resulting antibody designated as 44H4-Hu-IgG1.

**Example 2. Sources and Sequences of Anti-CD3 Monoclonal Antibody**

The amino acid sequences of the heavy chain and light chain variable regions of the murine anti-human CD3 monoclonal antibody (abbreviated as SP34) were derived from SEQ ID NO: 2 and 4 in US8236308B2, corresponding to SEQ ID NO: 15 and 16 in the present invention, respectively.

Amino acid sequence (SEQ ID NO: 15) of SP34 heavy chain variable region:

EVKLLESGGGLVQPKGSLKLSCAASGFTFNTYAMNWVRQAPGKGLEWVARIRSKYNN
YATYYADSVKDRFTISRDDSQSILYLQMNNLKTEDTAMYYCVRHGNFGNSYVSWFAY
WGQGTLVTVSA

Amino acid sequence (SEQ ID NO: 16) of SP34 light chain variable region:

QAVVTQESALTTSPGETVTLTCRSSTGAVTTSNYANWVQEKPDHLFTGLIGGTNKRAP
GVPARFSGSLIGDKAALTITGAQTEDEAIYFCALWYSNLWVFGGGTKLTVL

The amino acid sequences of the SP34 heavy and light chain variable regions were analyzed; and the complementarity determining regions (CDRs) and framework regions of the SP34 heavy and light chain variable regions were determined according to the Kabat numbering scheme, respectively.

The amino acid sequences of the three CDRs of the SP34 heavy chain were respectively as follows: H-CDR1: TYAMN (SEQ ID NO: 17), H-CDR2: RIRSKYNNYATYYADSVKD (SEQ ID NO: 18), and H-CDR3: HGNFGNSYVSWFAY (SEQ ID NO: 19), and the amino acid sequences of the three CDRs of the light chain were respectively as follows: L-CDR1: RSSTGAVTTSNYAN (SEQ ID NO: 20), L-CDR2: GTNKRAP (SEQ ID NO: 21), and L-CDR3: ALWYSNLWV (SEQ ID NO: 22).

The humanization process of the murine SP34 is described as follows. The heavy chain variable region of murine monoclonal antibody SP34 was compared with human antibody heavy chain variable region germline sequences for homology analysis. IGHV3-23*04 was selected as the humanization template. The three heavy chain CDRs of murine SP34 were grafted into IGHV3-23*04, replacing the corresponding CDRs of IGHV3-23*04, and the fourth framework region was added after H-CDR3 to obtain the CDR-grafted heavy chain variable region amino acid sequence. Similarly, the light chain variable region of SP34 was compared with human antibody light chain variable region germline sequences for homology analysis. IGLV7-46*01 was selected as the light chain CDR grafting template. The three light chain CDRs of murine SP34 were grafted into IGLV7-46*01, replacing the corresponding CDRs of IGLV7-46*01, and the fourth framework region was added after L-CDR3 to obtain the CDR-grafted light chain variable region amino acid sequence. On the basis of CDR grafted variable regions, reverse mutations were carried out on some sites in the framework regions. During the reverse mutation, the amino acid sequence was coded with Kabat, and the position of the site was indicated by the Kabat code.

Preferably, for the CDR-grafted heavy chain variable region, N at position 73 was reversely mutated to D, and K at position 94 was mutated to R. For the CDR-grafted light chain variable region, F at position 36 was mutated to V, T at position 46 was mutated to G, Y at position 49 was mutated to G, W at position 57 was mutated to G, and T at position 58 was mutated to V. In addition, A at position 2 of the CDR-grafted heavy chain variable region was mutated to T, and G at position 96 (in H-CDR3) was mutated to D. In addition, for the CDR-grafted light chain variable region, R at position 24 was mutated to G, A at position 33 was mutated to P, and N (in L-CDR3) at position 94 was mutated to D. The above modifications can further enhance the affinity, stability, and other properties of the SP34 humanized antibody. The aforementioned heavy and light chain variable regions with mutations were defined as the SP34 humanized heavy chain variable region (SEQ ID NO: 23) and light chain variable region (SEQ ID NO: 24), respectively.

DNAs encoding the aforementioned humanized heavy and light chain variable regions were synthesized by Sangon Biotech (Shanghai) Co., Ltd. Using genetic engineering methods, the synthesized humanized heavy chain variable region was fused with the human IgG1 heavy chain constant region (SEQ ID NO: 11) to obtain the full-length humanized heavy chain, designated as SP34-Hu-HC (SEQ ID NO: 25). Similarly, the humanized light chain variable region was fused with the human Lambda light chain constant region (SEQ ID NO: 26) to obtain the full-length humanized light chain, designated as SP34-Hu-LC (SEQ ID NO: 27). The coding genes of SP34-Hu-HC and SP34-Hu-LC were cloned into pcDNA3.4 expression vectors respectively, and the resulting heavy chain and light chain expression vectors were co-transfected into FreeStyle™ 293-F cells using PEI for antibody expression. After 5 days, the culture supernatant of FreeStyle™ 293-F cells was collected, and the antibody was purified from the supernatant by Protein A affinity chromatography, with the obtained antibody designated as SP34-Hu-IgG1.

## Example 3. Sources and Sequences of Anti-CD28 Monoclonal Antibody

The amino acid sequences of the heavy and light chain variable regions of the anti-human CD28 monoclonal antibody (referred to as Anti-CD28) were derived from SEQ ID NOs: 46 and 48 in US20060286104A1, corresponding to SEQ ID NOs: 28 and 29 in the present invention, respectively.

Amino acid sequence (SEQ ID NO: 28) of Anti-CD28 heavy chain variable region:

QVQLVQSGAEVKKPGASVKVSCKASGYTFTSYYIHWVRQAPGQGLEWIGCIYPGNVN TNYNEKFKDRATLTVDTSISTAYMELSRLRSDDTAVYFCTRSHYGLDWNFDVWGQGT TVTVSS

Amino acid sequence (SEQ ID NO: 29) of Anti-CD28 light chain variable region:

DIQMTQSPSSLSASVGDRVTITCHASQNIYVWLNWYQQKPGKAPKLLIYKASNLHTGV PSRFSGSGSGTDFTLTISSLQPEDFATYYCQQGQTYPYTFGGGTKVEIK

The amino acid sequences of the Anti-CD28 heavy and light chain variable regions were analyzed; and the complementarity determining regions (CDRs) and framework regions of the Anti-CD28 heavy and light chain variable regions were determined according to the Kabat numbering scheme, respectively. The amino acid sequences of the three CDRs of the Anti-CD28 heavy chain were respectively as follows: H-CDR1: SYYIH (SEQ ID NO: 30), H-CDR2: CIYPGNVNTNYNEKFKD (SEQ ID NO: 31), and H-CDR3: SHYGLDWNFDV (SEQ ID NO: 32), and
the amino acid sequences of the three CDRs of the light chain were respectively as follows: L-CDR1: HASQNIYVWLN (SEQ ID NO: 33), L-CDR2: KASNLHT (SEQ ID NO: 34), and L-CDR3: QQGQTYPYT (SEQ ID NO: 35).

To further enhance the humanization degree of the anti-CD28 monoclonal antibody, the amino acid sequence of its heavy chain variable region was modified, with the process described as follows. The amino acid sequence of the Anti-CD28 heavy chain variable region was aligned with the germline sequence of the heavy chain variable region of a human antibody for homology, and IGHV1-46*01 was selected as a reference template. Here, some amino acid residues in the framework regions of the Anti-CD28 heavy chain variable region were mutated to the amino acid residues in the IGHV1-46*01 framework regions at the corresponding positions. During the mutation, the amino acid sequence was coded with Kabat, and the position of the site was indicated by the Kabat code.

Preferably, for the amino acid sequence of the Anti-CD28 heavy chain variable region, I at position 48 was mutated to M, A at position 67 was mutated to V, and F at position 91 was mutated to Y. In addition, the first amino acid residue C in the H-CDR2 of the Anti-CD28 heavy chain variable region was mutated to S, to prevent the formation of covalent disulfide bonds between antibody molecules during production.

The amino acid sequence of modified Anti-CD28 heavy chain variable region is as follows:

QVQLVQSGAEVKKPGASVKVSCKASGYTFTSYYIHWVRQAPGQGLEWMGSIYPGNV NTNYNEKFKDRVTLTVDTSISTAYMELSRLRSDDTAVYYCTRSHYGLDWNFDVWGQG TTVTVSS (SEQ ID NO. 50)

DNAs encoding the aforementioned modified Anti-CD28 heavy chain variable region and unmodified Anti-CD28 light chain variable region were synthesized by Sangon Biotech (Shanghai) Co., Ltd. Using genetic engineering methods, the synthesized humanized heavy chain variable region was fused with the human IgG1 heavy chain constant region (SEQ ID NO: 11) to obtain the full-length humanized heavy chain, designated as Anti-CD28-Hu-HC (SEQ ID NO: 36). Similarly, the Anti-CD28 light chain variable region was fused with the human kappa light chain constant region (SEQ ID NO: 13) to obtain the full-length humanized light chain, designated as Anti-CD28-Hu-LC (SEQ ID NO: 37).

The coding genes of Anti-CD28-Hu-HC and Anti-CD28-Hu-LC were cloned into pcDNA3.4 expression vectors, respectively; the heavy and light chain expression vectors were co-transfected into FreeStyle™ 293-F cells using PEI for antibody expression; after 5 days, a FreeStyle™ 293-F cell culture supernatant was collected; and the antibody was purified from the supernatant by Protein A affinity chromatography, with the resulting antibody designated as Anti-CD28-Hu-IgG1.

**Example 4. Construction and Preparation of Anti-MUC17*CD3*CD28 Trispecific Antibody**

A first structure comprises three polypeptide chains with different sequences, with each chain encoded by a separate gene. Their design and construction methods are described as follows.

The method for constructing the first polypeptide chain or gene is described as follows:
the light chain variable region of Anti-CD28-Hu-IgG1 (containing a Q100C mutation) is linked to the heavy chain variable region of SP34-Hu-IgG1 via a short artificial linker (which is $G_4S$ here) by genetic engineering methods, with the terminus linked to the light chain variable region of SP34-Hu-IgG1 via a long artificial linker (which includes four $G_4S$ in tandem here), then linked to the heavy chain variable region of Anti-CD28-Hu-IgG1 (containing a G44C mutation) via a short artificial

linker (which is G$_4$S here), and then further linked to the hinge region and Fc fragment (which represents the CH2 and CH3 domains of the human IgG1 heavy chain constant region) of human IgG1 via a long artificial linker (which includes three G$_4$S in tandem here).

Here, the Fc fragment comprises L234A/L235A/G237A mutations to attenuate the interaction between Fc and Fcγ receptors (Reference: Liu R, Oldham R J, Teal E, et al. Fc-engineering for modulated effector functions - improving antibodies for cancer treatment[J]. Antibodies, 2020, 9(4): 64.), and also comprises S354C/T366W mutations to form a heterodimer of a Knob-Into-Hole (KIH) structure with another heavy chain polypeptide described later (Reference: Ha J H, Kim J E, Kim Y S. Immunoglobulin Fc heterodimer platform technology: from design to applications in therapeutic antibodies and proteins[J]. Frontiers in Immunology, 2016, 7: 394.).

The first polypeptide chain or gene is designated as CD28-44-SP34-Dia-IgG1-Knob-HC (with the amino acid sequence of SEQ ID NO: 38 and the nucleotide sequence of SEQ ID NO: 39). The method for constructing the second polypeptide chain or gene is described as follows:

the heavy chain variable region of 44H4-Hu-IgG1 is linked to a human IgG1 heavy chain constant region (including CH1, CH2, and CH3 domains) with many mutations. Here, the Fc fragment comprises L234A/L235A/G237A mutations to attenuate the interaction between Fc and Fcγ receptors, and also comprises Y349C/T366S/L368A/Y407V mutations to form a heterodimer of a KIH structure with another heavy chain polypeptide as described above. The second polypeptide chain or gene is designated as 44H4-IgG1-Hole-HC (with the amino acid sequence of SEQ ID NO: 40 and the nucleotide sequence of SEQ ID NO: 41).

The third polypeptide chain or gene, namely the 44H4-Hu-IgG1 light chain, remains unmodified and unaltered.

The coding genes of CD28-44-SP34-Dia-IgG1-Knob-HC, 44H4-IgG1-Hole-HC and 44H4-Hu-LC were cloned into pcDNA3.4 expression vectors, respectively; the expression vectors were co-transfected into FreeStyle™ 293-F cells using PEI for antibody expression; after 5 days, a FreeStyle™ 293-F cell culture supernatant was collected; and the antibody was purified from the supernatant by Protein A affinity chromatography, with the resulting antibody designated as CD28-44/SP34-Dia+44H4-KIH.

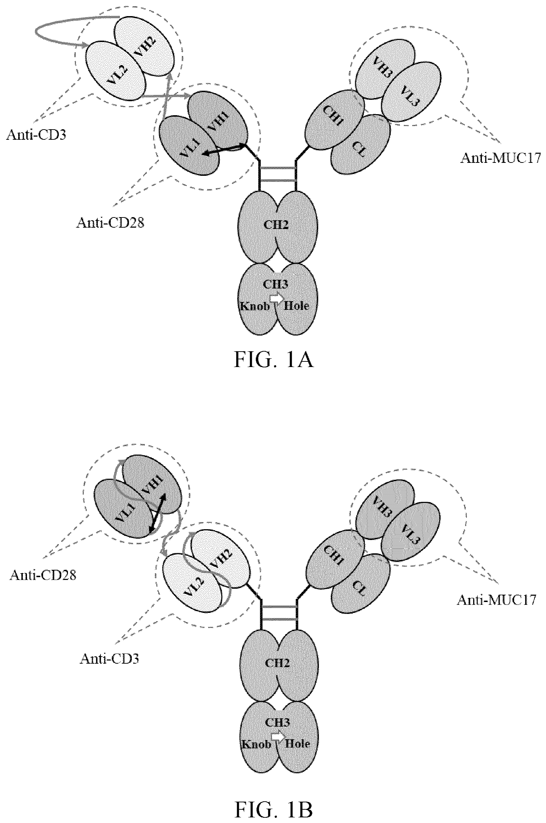

The schematic structural diagram of the trispecific antibody is shown in FIG. 1A, in which VL1 and VH1 represent the light and heavy chain variable regions of the anti-CD28 antibody, respectively, VH2 and VL2 represent the heavy and light chain variable regions of the anti-CD3 antibody, respectively, and VH3 and VL3 represent the heavy and light chain variable regions of the anti-MUC17 antibody, respectively. The straight and curved arrows indicate the artificially designed engagers and their directions, while the double-headed straight arrows indicate the positions of artificially designed disulfide bonds. VL1, VH2, VL2, and VH1 in FIG. 1A are sequentially attached by engagers of varying lengths, and ultimately folded and assembled into the structure of a Diabody (for the structure of Diabody, refer to Reference: Wu C. Diabodies: molecular engineering and therapeutic applications[J]. Drug News Perspect, 2009, 22(8): 453.).

The second structure comprises three polypeptide chains with different sequences, with each chain encoded by a separate gene. Their design and construction methods are described as follows.

The method for constructing the first polypeptide chain or gene is described as follows:

the light chain variable region of Anti-CD28-Hu-IgG1 (containing a Q100C mutation) is linked to the heavy chain variable region (containing a G44C mutation) of Anti-CD28-Hu-IgG1 via a long artificial linker (which includes four G$_4$S in tandem here) by genetic engineering methods, with the terminus linked to the light chain variable region of SP34-Hu-IgG1 via a short artificial linker (which includes two G$_3$S in tandem here), then linked to the heavy chain variable region of SP34-Hu-IgG1 via a long artificial linker (which includes four G$_4$S in tandem here), and then further linked to the hinge region and Fc fragment (which represents the CH2 and CH3 domains of the human IgG1 heavy chain constant region) of human IgG1 via a short artificial linkers (which includes two GGGS in tandem here). Here, the Fc fragment comprises L234A/L235A/-G237A mutations to attenuate the interaction between Fc and Fcγ receptors, and also comprises S354C/T366W mutations to form a heterodimer of a KIH structure with another heavy chain polypeptide described later. The first polypeptide chain or gene is designated as CD28-44-SP34-2ScFv-IgG1-Knob-HC (with the amino acid sequence of SEQ ID NO: 42 and the nucleotide sequence of SEQ ID NO: 43).

The second polypeptide chain or gene is 44H4-IgG1-Hole-HC.

The third polypeptide chain or gene is 44H4-Hu-LC.

The coding genes of CD28-44-SP34-2ScFv-IgG1-Knob-HC, 44H4-IgG1-Hole-HC and 44H4-Hu-LC were cloned into pcDNA3.4 expression vectors, respectively; the expression vectors were co-transfected into FreeStyle™ 293-F cells using PEI for antibody expression; after 5 days, a FreeStyle™ 293-F cell culture supernatant was collected; and the antibody was purified from the supernatant by Protein A affinity chromatography, with the resulting antibody designated as CD28-44/SP34-ScFv+44H4-KIH.

The schematic structural diagram of the trispecific antibody is shown in FIG. 1B, in which VL1 and VH1 represent the light and heavy chain variable regions of the anti-CD28 antibody, respectively, VL2 and VH2 represent the light and heavy chain variable regions of the anti-CD3 antibody, respectively, and VH3 and VL3 represent the heavy and light chain variable regions of the anti-MUC17 antibody, respectively. The curved arrows indicate the artificially designed engagers and their directions, while the double-headed straight arrows indicate the positions of artificially designed disulfide bonds.

VL1, VH1, VL2 and VH2 in FIG. 1B are sequentially attached by engagers of varying lengths, and ultimately folded and assembled into the structure of two ScFvs in tandem (for the structure of ScFv, refer to Reference: Ahmad Z A, Yeap S K, Ali A M, et al. scFv antibody: principles and clinical application[J]. Clinical and developmental immunology, 2012, 2012:980250.).

In this example, the mutations were introduced into the Fc fragment of the human IgG1 heavy chain constant region to construct a heavy chain constant region with a "knob" structure and a heavy chain constant region with a "hole" structure, respectively. H at position 435 of the "hole" chain was mutated to R and Y at position 436 was mutated to F, such that the binding effect of the "hole" chain to Protein A was eliminated, allowing the removal of the homodimer of the "hole" chain in the purification step (see Reference: Smith E, Olson K, Haber L, et al. A novel, native-format bispecific antibody triggering T-cell killing of B-cells is robustly active in mouse tumor models and cynomolgus monkeys[J]. Scientific Reports, 2016, 5(1): 17943-17943.). The Fc fragments of the above "knob"-structured heavy chain constant region and the modified "hole"-structured heavy chain constant region were designated as IgG1-Knob-HC (SEQ ID NO: 44) and IgG1-Hole-HC (SEQ ID NO:45), respectively.

### Example 5. Antigen Binding Capacity of Anti-MUC17*CD3*CD28 Trispecific Antibody

Methods for expressing and purifying relevant recombinant proteins are described as follows: the amino acid sequence of human MUC17 was sourced from Uniprot (Entry: Q685J3); the amino acid sequence from positions 4131 to 4390 was selected; a signal peptide was introduced at the N-terminus, and an engager ($G_4S$) along with six histidine residues were introduced at the C-terminus; the coding gene for the aforementioned recombinant amino acid sequence was obtained through gene synthesis; the coding gene of this recombinant protein was cloned into a pcDNA3.4 expression vector (purchased from Thermo Fisher Scientific); the expression vector was then transfected into FreeStyle™ 293-F cells (purchased from Thermo Fisher Scientific) using PEI to express the recombinant protein; and after an appropriate period, a cell culture supernatant was collected and filtered, followed by purification of the corresponding recombinant protein from the supernatant by nickel affinity chromatography, with the resulting recombinant protein designated as MUC17-His (SEQ ID: 46). The amino acid sequence of human CD28 was sourced from Uniprot (Entry: P10747); the amino acid sequence from positions 19 to 152 was selected; a signal peptide was introduced at the N-terminus, and an engager ($G_4S$) along with six histidine residues were introduced at the C-terminus; the coding gene for the aforementioned recombinant amino acid sequence was obtained through gene synthesis; and the recombinant protein was expressed and purified with a similar method as described above, with the resulting recombinant protein designated as CD28-His (SEQ ID: 47). In this example, the binding capacity of the anti-MUC17*CD3*CD28 trispecific antibody to MUC17, CD3, and CD28 was tested by ELISA (Enzyme-Linked Immunosorbent Assay) was used to determine, with a method described as follows.

The microplate (96-well) was coated with recombinant proteins MUC17-His, CD3-Epsilon (purchased from ACROBiosystems, Cat. No.: CDE-H5223) and CD28-His, respectively, with a coating amount of 10 ng/well for each recombinant protein; and the microplate was then blocked with PBST (which refers to phosphate-buffered saline containing 0.05% Tween-20) containing 1% bovine serum albumin. The antibody under test was subjected to gradient dilution, then transferred to the microplate coated with recombinant proteins as described above, and then incubated at room temperature for half an hour, followed by plate washing; an appropriately diluted HRP (Horseradish Peroxidase)-labeled goat anti-human antibody (Fc specific, purchased from Sigma, Cat. No.: SAB3701283) was added, followed by incubation at room temperature for half an hour and subsequent plate washing; 100 μL of a chromogenic solution using TMB (3,3',5,5'-Tetramethylbenzidine) as a substrate was added to each well, which was then incubated at room temperature for 1 to 5 minutes; 50 μL of a stop solution (2M $H_2SO_4$) was added to stop the reaction; and OD450 was read using a Microplate Reader (model: SpectraMax 190), and plotting and data analysis was carried out using GraphPad Prism7 to calculate $EC_{50}$ (concentration for 50% of maximal effect).

Table 1. Antigen Binding Capacity of Anti-MUC17*CD3*CD28 Trispecific Antibody

| Antibody | $EC_{50}$ (nM) | | |
|---|---|---|---|
| | MUC17-His | CD3-Epsilon | CD28-His |
| 44H4-Hu-IgG 1 | 0.06507 | NA | NA |
| SP34-Hu-IgG1 | NA | 0.05533 | NA |
| Anti-CD28-Hu-IgG1 | NA | NA | 0.1166 |
| CD28-44/SP34-Dia+44H4-KIH | 0.06246 | 0.9199 | 0.8207 |
| CD28-44/SP34-ScFv+44H4-KIH | 0.1732 | 3.363 | 1.711 |

**EP 4 682 172 A1**

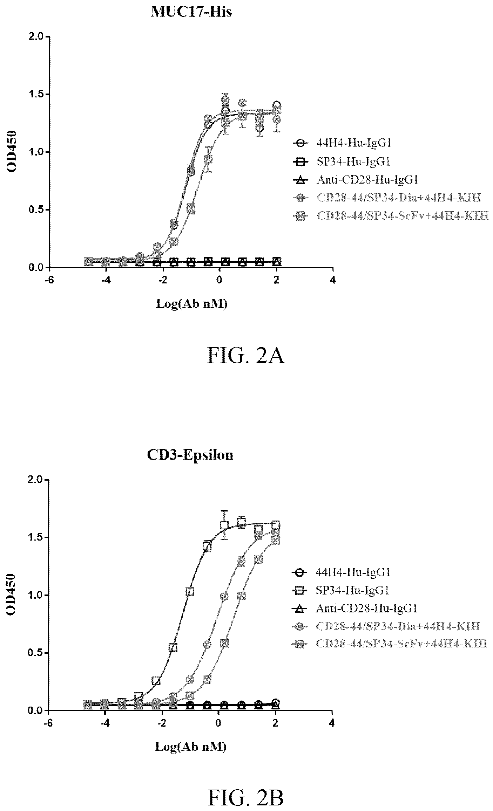

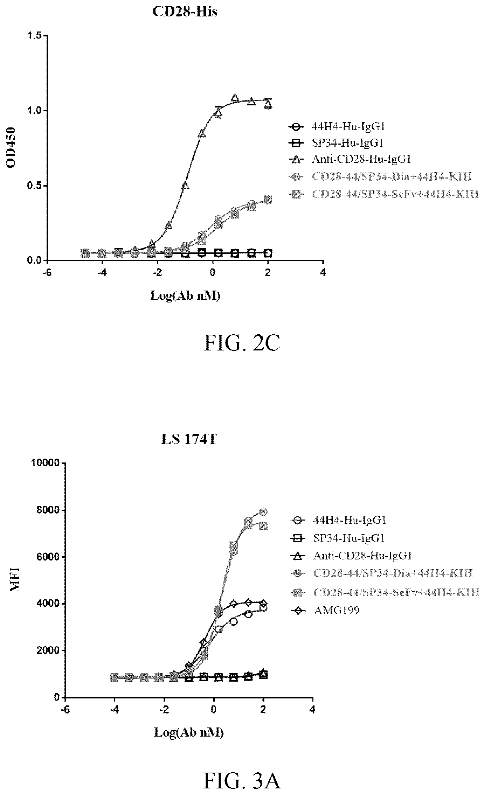

Table 1 summarizes the calculation results for each curve in FIGs. 2A, 2B and 2C. As shown in FIGs. 2A, 2B and 2C and Table 1, 44H4-Hu-IgG1, SP34-Hu-IgG1 and Anti-CD28-Hu-IgG1 can effectively bind to MUC17-His, CD3-Epsilon and CD28-His, respectively, with $EC_{50}$ values of 0.06507 nM, 0.05533 nM, and 0.1166 nM. CD28-44/SP34-Dia+44H4-KIH can effectively bind to MUC17-His, CD3-Epsilon and CD28-His, with $EC_{50}$ values of 0.06246 nM, 0.9199 nM, and 0.8207 nM, respectively. CD28-44/SP34-ScFv+44H4-KIH can also effectively bind to MUC17-His, CD3-Epsilon, and CD28-His, with $EC_{50}$ values of 0.1732 nM, 3.363 nM, and 1.711 nM, respectively.

Both CD28-44/SP34-Dia+44H4-KIH and CD28-44/SP34-ScFv+44H4-KIH can effectively bind to MUC17-His, CD3-Epsilon, and CD28-His, indicating that they are trispecific antibodies.

**Example 6. Binding Capacity of Anti-MUC17*CD3*CD28 Trispecific Antibody to Cell Surface Antigens**

The binding capacity of the anti-MUC17*CD3*CD28 trispecific antibody to MUC17 on the surface of human colon adenocarcinoma cells LS174T (purchased from ATCC, Cat. No.: CL-188), as well as to CD3/CD28 on the surface of human T cells was tested using the flow cytometer.

The specific method is described as follows: LS 174T cells or fresh human peripheral blood mononuclear cells (referred to as PBMCs, purchased from Shanghai OriBiotech Co., Ltd., Cat. No.: FPB002-C-200) were seeded into a 96-well round-bottom microplate (300,000 cells per well) and centrifuged at 300 g for 5 minutes; the supernatant was removed using a multichannel pipette; 200 μL of phosphate-buffered saline (PBS) containing 1% bovine serum albumin (BSA) (denoted as PBS+1% BSA) was added to to each well to resuspend the cell pellet; and the the microplate was centrifuged at 300 g for 5 minutes, followed by removal of the supernatant. The gradient-diluted antibodies were added; the cell pellets were resuspended; and the microplate was incubated at room temperature for about half an hour. The microplate was centrifuged at 300 g for 5min, followed by removal of the supernatant, and the cells in each well were washed twice with 200 μL of PBS. An appropriate amount of R-phycoerythrin-labeled goat anti-human IgG (purchased from Jackson ImmunoResearch, Cat. No.: 109-115-098) diluted at 1:1000 with PBS + 1% BSA was added to each well to resuspend the cell pellet, and the microplate was incubated at room temperature for about half an hour. For PBMCs, APC mouse anti-Human CD4 (purchased from BD Biosciences, Cat. No.: 555349) was also added to label CD4-positive T cells. The microplate was centrifuged at 300 g for 5min, followed by removal of the supernatant, and the cells in each well were washed twice with 200 μL of PBS. The fix buffer I (purchased from BD Biosciences, Cat. No.: 557870) was added to each well to fix the cells, which were than incubated at room temperature for 5 minutes. The microplate was centrifuged at 300 g for 5min, followed by removal of the supernatant, and the cells in each well were washed twice with 200 μL of PBS and finally resuspended in 200 μL of PBS. The fluorescence intensity in the PE channel was measured using the flow cytometer, CytoFLEX Cytometer System (purchased from Beckman Coulter). For PBMCs, the PE fluorescence intensity was tested here for CD4-positive T cells. The data were processed using flow cytometer software to export the mean fluorescence intensity (MFI) of the PE channel for each sample. Data analysis and plotting were carried out using GraphPad Prism 7 to calculate the $EC_{50}$.

Here, the anti-MUC17 and anti-CD3 bispecific antibody AMG199 developed by Amgen was introduced as a control antibody. The amino acid sequence of AMG199 was sourced from SEQ ID NO: 171 in WO2022060901A1. AMG199 was prepared following the methods for expression and purification as described in the above examples.

Table 2. Binding capacity of anti-MUC17*CD3*CD28 trispecific antibody to cell surface antigens

| Antibody | LS 174T | | CD4 T | |
|---|---|---|---|---|
| | $EC_{50}$ (nM) | Top | $EC_{50}$ (nM) | Top |
| 44H4-Hu-IgG 1 | 0.652 | 3745 | NA | NA |
| SP34-Hu-IgG1 | NA | NA | 0.6691 | 39823 |
| Anti-CD28-Hu-IgG1 | NA | NA | 2.38 | 21894 |
| CD28-44/SP34-Dia+44H4-KI H | 2.265 | 8072 | 4.175 | 31073 |
| CD28-44/SP34-ScFv+44H4-KI H | 1.844 | 7494 | 2.802 | 26579 |
| AMG199 | 0.4247 | 4068 | 2.714 | 12929 |

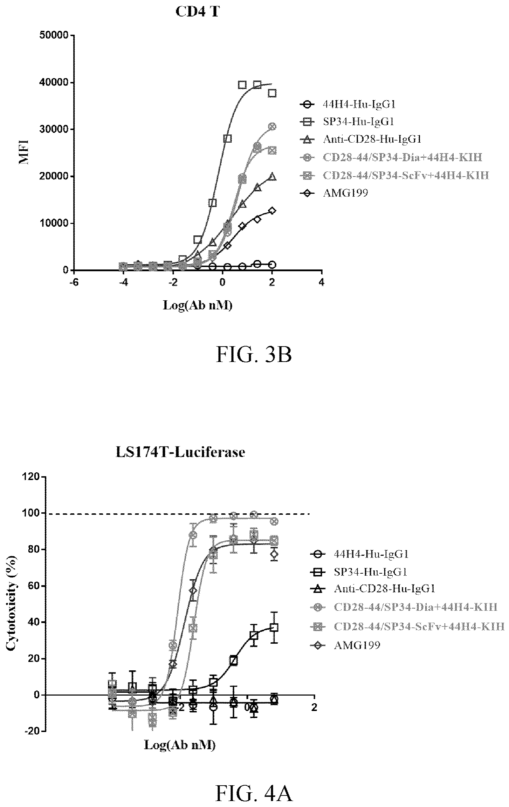

Table 2 summarizes the calculation results for each curve in FIGs. 3A and 3B. Top represents the high plateau of the fitted curve, indicating the theoretical maximum signal intensity of antibody binding.

As shown in FIG. 3A and Table 2, 44H4-Hu-IgG1, CD28-44/SP34-Dia+44H4-KIH, CD28-44/SP34-ScFv+44H4-KIH and AMG199 all can effectively bind to LS 174T cells, with $EC_{50}$ values of 0.652 nM, 2.265 nM, 1.844 nM, and 0.4247 nM, respectively, and Top values of 3745, 8072, 7494, and 4068, respectively.

19

The results above show that the binding capacities of 44H4-Hu-IgG1 and AMG199 to LS 174T cells are comparable, and the binding capacities of CD28-44/SP34-Dia+44H4-KIH and CD28-44/SP34-ScFv+44H4-KIH to LS 174 T cells are also comparable. At high concentrations, the Top values of CD28-44/SP34-Dia+44H4-KIH and CD28-44/SP34-ScFv+44H4-KIH are significantly higher than those of 44H4-Hu-IgG1 and AMG199, indicating that the trispecific antibody of the present invention exhibits a superior binding capacity to cell surface antigens as compared to the latter two.

As shown in FIG. 3B and Table 2, SP34-Hu-IgG1, Anti-CD28-Hu-IgG1, CD28-44/SP34-Dia+44H4-KIH, CD28-44/SP34-ScFv+44H4-KIH and AMG199 all can effectively bind to CD4-positive T cells, with $EC_{50}$ values of 0.6691 nM, 2.38 nM, 4.175 nM, 2.802 nM, and 2.714 nM, respectively, and Top values of 39823, 21894, 31073, 26579, and 12929, respectively.

The above results show that the monoclonal antibody SP34-Hu-IgG1 exhibits the strongest binding capacity to CD4 T cells, Anti-CD28-Hu-IgG1, CD28-44/SP34-Dia+44H4-KIH and CD28-44/SP34-ScFv+44H4-KIH show comparable binding capacities to CD4 T cells, and AMG199 shows the weakest binding capacity.

**Example 7. Cytotoxicity of Anti-MUC17\*CD3\*CD28 Trispecific Antibody Against Target Cells**

The method for preparing target cells is described as follows: the sequence of a Luciferase gene was sourced from GenBank (MK484108.1), its coding gene was synthesized by Sangon Biotech (Shanghai) Co., Ltd., and the luciferase gene was cloned into the pLVX-puro lentiviral vector (purchased from Clontech, Cat. No.: 632164), with the resulting expression vector designated as pLVX-Luciferase-puro. Lentiviral packaging plasmids and the pLVX-Luciferase-puro were co-transfected into HEK293 cells to prepare recombinant lentiviruses carrying the luciferase gene. A cell culture supernatant containing recombinant lentiviruses was collected to infect LS 174T cells, and after three days, pressurized screening was carried out with puromycin to obtain clones of cells transfected with the target genes. The final cell strains stably expressing luciferase were designated as LS174T-Luciferase.

CD3-positive T cells (purchased from Shanghai OriBiotech Co., Ltd., Cat. No.: FPB009-1-C-100) were used as effector cells here. Under the mediation of the anti-MUC17\*CD3\*CD28 trispecific antibody, these effector cells were effectively activated to kill the above established tumor target cells LS174T-Luciferase. The killing effect of the effector cells or the degree of killing the target cells were reflected by measuring the intensity of luciferase, thereby calculating the functional activity of the trispecific antibody.

LS174T-Luciferase and CD3-positive T cells were mixed at a ratio of 1:10 and centrifuged to discard the supernatant; then, the cell pellet was resuspended in an Advanced RPMI 1640 complete medium (containing 10% FBS, 1% penicillin streptomycin, 1% GlutaMAX, and 1‰ 2-mercaptoethanol); and subsequently, the cell mixture was seeded into a 96-well flat-bottom cell culture plate at a density of 10,000 LS174T-Luciferase cells per well. Then, the respective gradient-diluted antibodies were added. In addition, 0.01% Triton X-100 was added to several wells to completely kill the target cells, while the complete medium without antibodies was supplemented in several further wells. After 72 hours, the luciferase intensity was measured using the Bio-Glo™ luciferase assay system (purchased from Promega, Cat. No.: G7940). The mean luciferase intensity of several wells with the addition of Triton X-100 was used as the minimum background value, while the mean luciferase intensity of several wells without antibodies was used as the maximum reference value for cell proliferation. A formula for calculating antibody cytotoxicity is:

$$\text{Cytotoxicity} = (\text{Maximum reference value} - \text{Luciferase})/(\text{Maximum reference value} - \text{Minimum background value}) * 100\%.$$

Table 3A. Cytotoxicity of anti-MUC17\*CD3\*CD28 trispecific antibody against target cells

| Antibody | $EC_{50}$ (pM) | Top (%) |
|---|---|---|
| 44H4-Hu-IgG 1 | NA | NA |
| SP34-Hu-IgG 1 | 432.4 | 37.76 |
| Anti-CD28-Hu-IgG 1 | NA | NA |
| CD28-44/SP34-Dia+44H4-KIH | 8.418 | 97.11 |
| CD28-44/SP34-ScFv+44H4-KIH | 25.71 | 85.14 |
| AMG199 | 13.63 | 83.04 |

Table 3B. Cytotoxicity of anti-MUC17*CD3*CD28 trispecific antibody against target cells

| Antibody | $EC_{50}$ (pM) | Top (%) |
|---|---|---|
| 44H4-Hu-IgG 1 | NA | NA |
| SP34-Hu-IgG1 | 253.3 | 49.58 |
| Anti-CD28-Hu-IgG 1 | NA | NA |
| CD28-44/SP34-Dia+44H4-KIH | 9.284 | 93.55 |
| CD28-44/SP34-ScFv+44H4-KIH | 20.59 | 85.72 |
| AMG199 | 10.48 | 83.91 |

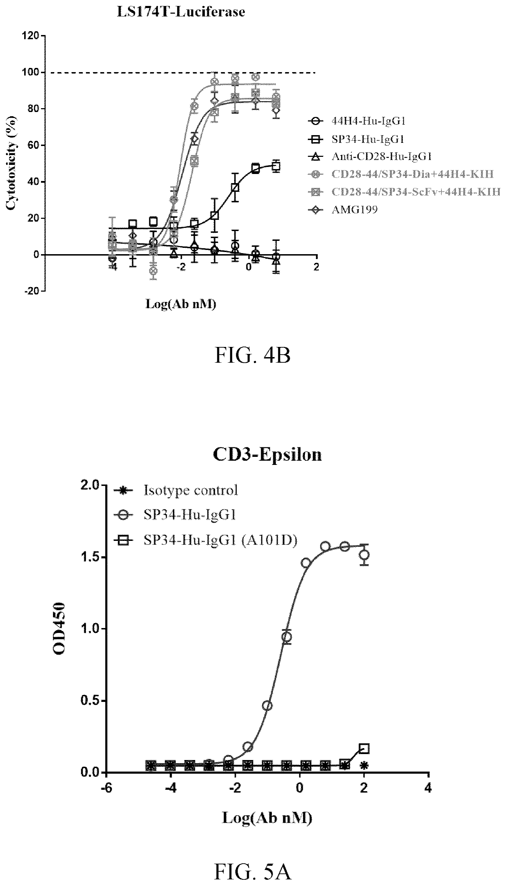

FIGs. 4A and 4B illustrate the results of two independent repeated experiments, in which effector cells used are sourced from different human individuals. Tables 3A and 3B summarize the calculated results for each curve in FIGs. 4A and 4B, respectively. "Top" represents the high plateau of the fitted curve, indicating the theoretical maximum signal intensity of cytotoxicity.

The results in FIG. 4A and Table 3A show that the monoclonal antibodies 44H4-Hu-IgG1 and Anti-CD28-Hu-IgG1 exhibit no cytotoxicity against the target cells LS174T-Luciferase, while SP34-Hu-IgG1 exhibits weak cytotoxicity ($EC_{50}$: 432.4 pM; Top: 37.76%). CD28-44/SP34-Dia+44H4-KIH, CD28-44/SP34-ScFv+44H4-KIH and AMG199 all can effectively kill LS174T-Luciferase cells, with $EC_{50}$ values of 8.418 pM, 25.71 pM, and 13.63 pM, respectively, and Top values of 97.11%, 85.14%, and 83.04%, respectively. CD28-44/SP34-Dia+44H4-KIH shows the highest degree of killing target cells (97.11%). Based on comprehensive evaluation, the ranking of the three antibodies in terms of their cytotoxicity against target cells, from strongest to weakest, was determined as follows: CD28-44/SP34-Dia+44H4-KIH > AMG199 > CD28-44/SP34-ScFv+44H4-KIH.

The results in FIG. 4B and Table 3B show that the monoclonal antibodies 44H4-Hu-IgG1 and Anti-CD28-Hu-IgG1 exhibit no cytotoxicity against the target cells LS174T-Luciferase, while SP34-Hu-IgG1 exhibits weak cytotoxicity ($EC_{50}$: 253.3 pM; Top: 49.58%). CD28-44/SP34-Dia+44H4-KIH, CD28-44/SP34-ScFv+44H4-KIH and AMG199 all can effectively kill LS174T-Luciferase cells, with $EC_{50}$ values of 9.284 pM, 20.59pM and 10.48pM, respectively, and Top values of 93.55%, 85.72% and 83.91%, respectively. CD28-44/SP34-Dia+44H4-KIH shows the highest degree of killing target cells (93.55%).

Based on comprehensive evaluation, the ranking of the three antibodies in terms of their cytotoxicity against target cells, from strongest to weakest, was determined as follows: CD28-44/SP34-Dia+44H4-KIH > AMG199 > CD28-44/SP34-ScFv+44H4-KIH.

## Example 8. Evaluation of Superiority of Anti-MUC17*CD3*CD28 Trispecific Antibody over Bispecific Antibodies

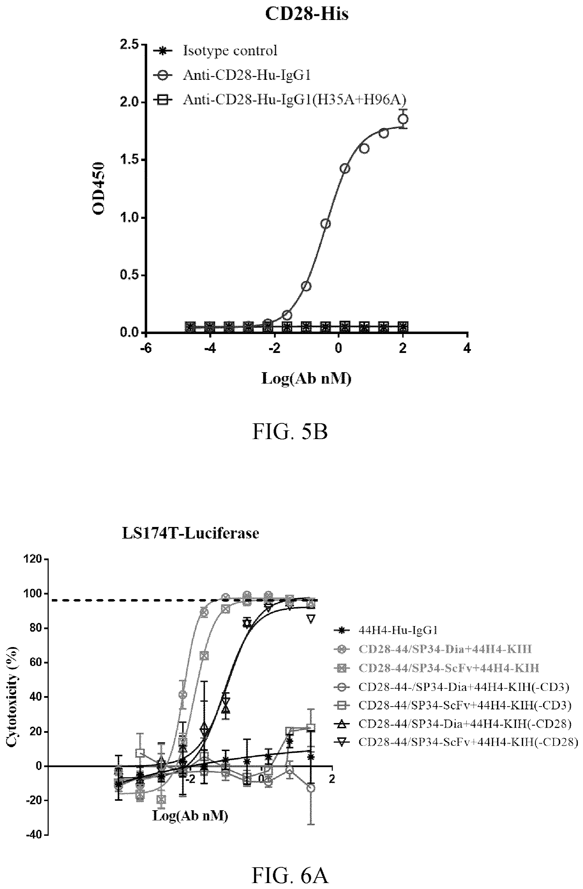

Site-directed mutations were introduced at key positions in the CDRs of monoclonal antibodies SP34-Hu-IgG1 and Anti-CD28-Hu-IgG1, respectively, to prepare a series of mutants for each antibody. The binding activity of these mutants was evaluated using the ELISA method described in Example 5 above, resulting in monoclonal antibody mutants that no longer bound to their respective targets. The results in FIG. 5A show that the introduction of an A101D mutation in the CDR3 of the SP34-Hu-IgG1 heavy chain results in the prepared mutant SP34-Hu-IgG1 (A101D) that no longer binds to CD3-Epsilon. The results in FIG. 5B show that the introductions of an H35A mutation in the CDR1 of the Anti-CD28-Hu-IgG1 heavy chain and an H96A mutation in the CDR3 result in the prepared mutant Anti-CD28-Hu-IgG1 (H35A+H96A) that no longer bound to CD28.

CD28-44/SP34-Dia+44H4-KIH and CD28-44/SP34-ScFv+44H4-KIH each comprise the VH and VL of SP34-Hu-IgG1, with an A101D mutation introduced in the VH; the mutated antibodies are re-prepared and designated as CD28-44/SP34-Dia+44H4-KIH (-CD3) and CD28-44/SP34-ScFv+44H4-KIH (-CD3), respectively; and these mutated antibodies are no longer capable of binding to CD3-Epsilon and are theoretically bispecific antibodies capable of binding to both MUC17 and CD28.

CD28-44/SP34-Dia+44H4-KIH and CD28-44/SP34-ScFv+44H4-KIH each comprise the VH and VL of Anti-CD28-Hu-IgG1, with H35A and H96A mutations introduced in the VH; the mutated antibodies are re-prepared and designated as CD28-44/SP34-Dia+44H4-KIH (-CD28) and CD28-44/SP34-ScFv+44H4-KIH (-CD28), respectively; and these mutated antibodies are no longer capable of binding to CD28 and are theoretically bispecific antibodies capable of binding to both MUC17 and CD3.

The method described in Example 7 was used to determine the cytotoxicity of the aforementioned trispecific antibody and it mutants against target cells. Table 4 summarizes the calculation results for each curve in FIG. 6A.

The results in FIG. 6A and Table 4 show that CD28-44/SP34-Dia+44H4-KIH and CD28-44/SP34-ScFv+44H4-KIH can still effectively kill target cells, with $EC_{50}$ values of 6.6 pM and 12.99 pM, respectively, and Top values of 97.51% and 96.41%, respectively. CD28-44/SP34-Dia+44H4-KIH (-CD3) and CD28-44/SP34-ScFv+44H4-KIH (-CD3) exhibit no cytotoxicity against the target cells. The $EC_{50}$ values of CD28-44/SP34-Dia+44H4-KIH (-CD28) and CD28-44/SP34-ScFv+44H4-KIH (-CD28) for the cytotoxicity against target cells are 117.8 pM and 87.59 pM, respectively, with Top values of 98.15% and 92.51%. Compared to the parental trispecific antibody, their mutants show significantly weaker cytotoxicity.

Table 4. Cytotoxicity of trispecific antibody and its mutants against LS174T-Luciferase cells

| Antibody | $EC_{50}$ (pM) | Top (%) |
|---|---|---|
| 44H4-Hu-IgG 1 | NA | NA |
| CD28-44/SP34-Dia+44H4-KIH | 6.6 | 97.51 |
| CD28-44/SP34-ScFv+44H4-KIH | 12.99 | 96.41 |
| CD28-44/SP34-Dia+44H4-KIH(-CD3) | NA | NA |
| CD28-44/SP34-ScFv+44H4-KIH(-CD3) | NA | NA |
| CD28-44/SP34-Dia+44H4-KIH(-CD28) | 117.8 | 98.15 |
| CD28-44/SP34-ScFv+44H4-KIH(-CD2 8) | 87.59 | 92.51 |

In the aforementioned cytotoxicity assay, the effector cells were CD3-positive T cells, and the target cells were LS174T-Luciferase cells. Under the mediation of the trispecific antibody or its mutants, T cells recognized specific antigens expressed on the surface of target cells and became activated; and the activated T cells secreted certain cytokines, which further activated T cells and enhanced their cytotoxicity. To further verify the superiority of the trispecific antibody over the bispecific antibody, the concentration of INF-gamma in the supernatant of the aforementioned cell-killing reaction system was also measured in this example. The method for determining the concentration of INF-gamma in the supernatant is described as follows.

An ELISA microplate was coated with anti-human IFN-gamma antibodies (BD Biosciences, Cat. No.: 551221) at a coating amount of 0.3 μg per well, and then blocked with PBST (which refers to phosphate-buffered saline containing 0.05% Tween-20) containing 1% bovine serum albumin. An IFN-gamma standard (BD Biosciences, Cat. No.: 554617) was formulated with PBST containing 1% BSA. The supernatant in the aforementioned cell-killing reaction system was diluted 4-fold with PBST containing 1% BSA. The gradient-diluted IFN-gamma standards and the 4-fold diluted supernatant were transferred into the microplate coated with the anti-human IFN-gamma antibodies at 100 μL per well, and incubated at room temperature for 1 hour; after the microplate was washed 3 times with PBST, 100 μL of biotinylated anti-human IFN-gamma antibodies (BD Biosciences, Cat. No.: 554550. Dilute 1000-fold with PBST containing 1% BSA before use) was added to each well, followed by incubation at room temperature for 0.5 hours; and after the microplate was washed 3 times with PBST, 100 μL of Streptavidin HRP (BD Biosciences, Cat. No.: 554066. Dilute 1000-fold with PBST containing 1% BSA immediately before use) was added to each well, followed by incubation at room temperature for 0.5 hours; after the microplate was washed 3 times with PBST, 100 μL of a chromogenic solution with TMB (3,3',5,5'-Tetramethylbenzidine) as a substrate was added to each well, followed by incubation at room temperature for 1 to 5 minutes; 50 μL of a stop solution (2M $H_2SO_4$) was added to stop the reaction; OD450 was read using a Microplate Reader (Model: SpectraMax 190) and the IFN-gamma concentration in each well was calculated based on the standard curve; and plotting and data analysis were carried out using GraphPad Prism7 to calculate $EC_{50}$.

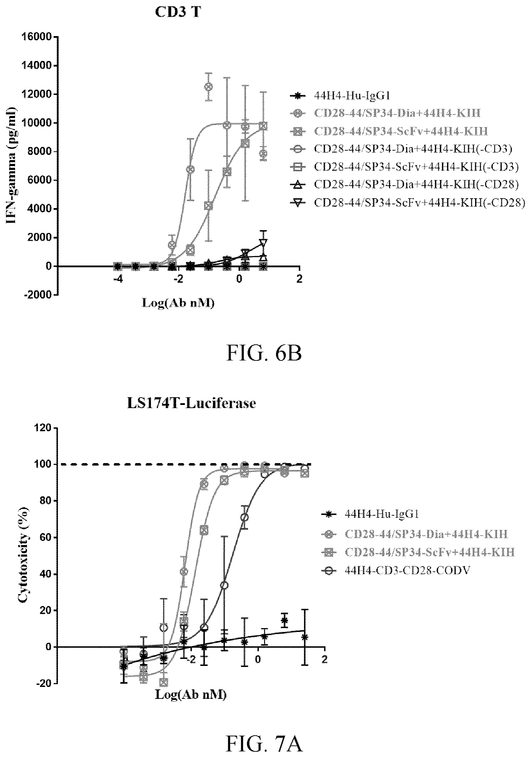

The results in FIG. 6B show that CD28-44/SP34-Dia+44H4-KIH and CD28-44/SP34-ScFv+44H4-KIH can effectively stimulate IFN-gamma secretion from CD3-positive T cells, with $EC_{50}$ values of 16.65 pM and 165.8 pM, respectively; and at high concentrations, the antibodies can induce IFN-gamma secretion at a level up to 10 ng/mL. CD28-44/SP34-Dia+44H4-KIH (-CD3) and CD28-44/SP34-ScFv+44H4-KIH (-CD3) exhibit no activity in promoting IFN-gamma secretion. Compared to the parental trispecific antibody, CD28-44/SP34-Dia+44H4-KIH (-CD28) and CD28-44/SP34-ScFv+44H4-KIH (-CD28) exhibit very weak activity in stimulating T cells to secrete IFN-gamma.

The results of this example show that the cytotoxicity of CD28-44/SP34-Dia+44H4-KIH and CD28-44/SP34-ScFv+44H4-KIH against target cells is correlated and consistent with their ability to stimulate T cells to secrete IFN-gamma.

CD28 is a classical costimulatory molecule expressed on the surface of T cells. The trispecific antibody of the present invention can further bind to CD28 while binding to CD3, thereby providing dual signals for T cell activation. Consequently, the trispecific antibody is more effective in activating the cytotoxicity against T cells as compared to the conventional bispecific antibodies.

**Example 9. Evaluation of Superiority of Anti-MUC17*CD3*CD28 Trispecific Antibodies over CODV-IgG**

CODV-Ig is Sanofi's multispecific antibody platform (Reference: Steinmetz A, Vallée F, Beil C, et al. CODV-Ig, a universal bispecific tetravalent and multifunctional immunoglobulin format for medical applications[C]//MAbs. Taylor & Francis, 2016, 8(5): 867-878.). With this platform, Sanofi has designed a trispecific antibody targeting HER2, CD3 and CD28 (Reference: Seung E, Xing Z, Wu L, et al. A trispecific antibody targeting HER2 and T cells inhibits breast cancer growth via CD4 cells[J]. Nature, 2022, 603(7900): 328-334.).

To demonstrate the superiority of the anti-MUC17*CD3*CD28 trispecific antibody of the present invention over CODV-IgG, an anti-MUC17*CD3*CD28 trispecific antibody with a CODV-IgG structure and the variable region sequences of a related anti-CD3 and anti-CD28 antibody was prepared in this example in a process described as follows.

The amino acid sequence of the heavy chain variable region of the CODV-IgG-related anti-CD3 and anti-CD28 antibody was sourced from SEQ ID: 64 in US20200140552A1; this sequence was spliced with the sequence of the heavy chain constant region of the aforementioned polypeptide CD28-44-SP34-Dia-IgG1-Knob-HC; and a coding gene encoding this recombinant sequence was obtained through genetic engineering methods, and this polypeptide and its coding gene were designated as CD28-CD3-CODV-IgG1-Knob-HC (SEQ ID: 48). The amino acid sequence of the light chain of the CODV-IgG-related anti-CD3 and anti-CD28 antibody was sourced from SEQ ID: 61 in US20200140552A1. Here, this polypeptide and its coding gene were designated as CD3-CD28-CODV-LC (SEQ ID: 49).

The coding genes of CD28-CD3-CODV-IgG1-Knob-HC and CD3-CD28-CODV-LC were cloned into pcDNA3.4 expression vectors, respectively; these expression vectors were combined with 44H4-IgG1-Hole-HC and 44H4-Hu-LC expression vectors in the aforementioned examples in proportion, and then co-transfected into FreeStyle™ 293-F cells using PEI for antibody expression; after 5 days, a FreeStyle™ 293-F cell culture supernatant was collected; and the antibody was purified from the supernatant by Protein A affinity chromatography, with the resulting antibody designated as 44H4-CD3-CD28-CODV.

In this example, a study and comparison of activities were carried out between the present invention and the CODV-IgG structure-based trispecific antibody; the cytotoxicity of these trispecific antibodies were measured using the methods described in the previous examples; and the secretion of IFN-gamma in the cell reaction system was also determined.

The results in FIG. 7A show that CD28-44/SP34-Dia+44H4-KIH and CD28-44/SP34-ScFv+44H4-KIH can still effectively kill target cells, with $EC_{50}$ values of 6.6 pM and 12.99 pM, respectively. The $EC_{50}$ value of 44H4-CD3-CD28-CODV for cytotoxicity against target cells was 173.2 pM. Compared to the two trispecific antibodies of the present invention, the activity of 44H4-CD3-CD28-CODV is significantly weaker.

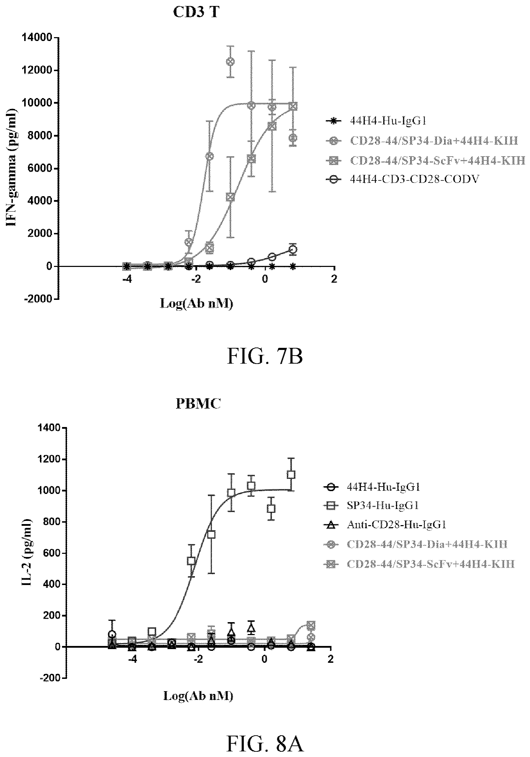

The results in FIG. 7B show that CD28-44/SP34-Dia+44H4-KIH and CD28-44/SP34-ScFv+44H4-KIH can effectively stimulate IFN-gamma secretion from CD3-positive T cells, with $EC_{50}$ values of 16.65 pM and 165.8 pM, respectively; and at high concentrations, the antibodies can induce IFN-gamma secretion at a level up to 10 ng/mL. Compared to the two trispecific antibodies of the present invention, 44H4-CD3-CD28-CODV exhibits very weak activity in stimulating T cells to secrete IFN-gamma.

**Example 10. Evaluation of In Vitro Safety of Anti-MUC17*CD3*CD28 Trispecific Antibodies**

PBMCs (purchased from Shanghai OriBiotech Co., Ltd., Cat. No.: FPB002-C-200) were washed twice with PBS; cell pellets were resuspended in an Advanced RPMI 1640 complete medium (containing 10% FBS, 1% penicillin streptomycin, 1% GlutaMAX, and 1‰ 2-mercaptoethanol); and After the cells were counted and adjusted in density, the cells were seeded into a 96-well round-bottom cell culture plate at 300,000 cells per well in 150 μL per well. In another 96-well microplate, the antibodies were diluted with the culture medium in a gradient, and then 50 μL of the gradient-diluted antibodies were added to the aforementioned cell culture plate. The 96-well cell culture plate containing the antibodies was incubated in a $CO_2$ incubator at 37°C for 2 days. An appropriate amount of cell culture supernatant was collected to measure the secretion volumes of IL-2 and IFN-gamma.

The method for measuring the secretion volume of IL-2 was very similar to the method for measuring IFN-gamma described in the aforementioned examples, and a double-antibody sandwich assay was also used, with relevant reagents purchased from BD Biosciences, including anti-human IL-2 antibodies (Catalog No.: 9133977), IL-2 standard (Catalog No.: 9220085), and biotinylated anti-human IL-2 antibodies (Catalog No.: 9120557). After the excess supernatant was discarded from the cell culture plate, the cell proliferation was measured using the CellTiter-Glo Luminescent Cell Viability Assay kit (purchased from Promega, Cat. No. G7572).

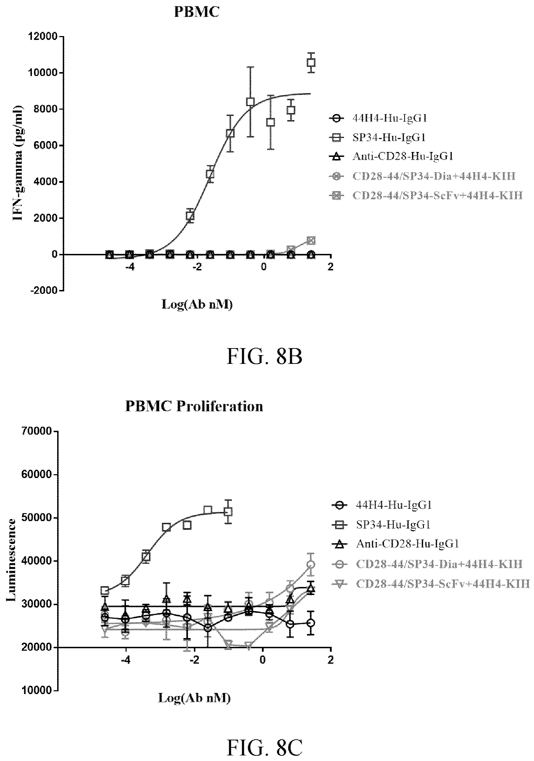

The experimental results show that the SP34-Hu-IgG1 monoclonal antibody strongly stimulates PBMCs to secrete IL-2 (FIG. 8A, $EC_{50}$: 7.596 pM) and IFN-gamma (FIG. 8B, $EC_{50}$: 24.49 pM), and also stimulates PBMC proliferation (FIG. 8C, $EC_{50}$: 0.4244 pM). The results of the above in vitro assay indicate that the two trispecific antibodies of the present invention do not non-specifically activate PBMC cells within a reasonable concentration range.

**Example 11. Evaluation of Physiochemical Properties of Anti-MUC17*CD3*CD28 Trispecific Antibody**

Antibodies are high-molecular-weight proteins with highly complex secondary and tertiary structures. Due to changes such as post-translational modifications, aggregation, and degradation, the antibodies are heterogeneous in their biochemical and biophysical properties. When bispecific antibodies are analyzed using separation techniques, variants, aggregates and degradation fragments are commonly observed, and their presence may compromise safety and efficacy. During the production and storage of antibodies, aggregates, degradation fragments and incompletely assembled molecules are prone to occur.

The present invention employs high-performance liquid chromatography-size exclusion chromatography (HPLC-SEC) to determine the content of the aforementioned impurities in samples. The molecular weight of aggregates is greater than that of monomers, resulting in a shorter retention time for the corresponding peak; and the molecular weight of degradation fragments or incompletely assembled molecules is smaller than that of monomers, resulting in a longer retention time for the corresponding peak. The HPLC-SEC system used was a Dionex Ultimate 3000 chromatograph; the mobile phases were prepared as follows: an appropriate amount of 20 mM sodium dihydrogen phosphate stock solution was taken and adjusted to pH $6.8 \pm 0.1$ using 20 mM disodium hydrogen phosphate; the injection volume was 20 $\mu$g; the column used was TSK G3000SWXL with the dimensions of $7.8 \times 300$ mm and the particle size of 5 $\mu$m; the flow rate was set at 0.5 mL/min with an elution time of 30 minutes; the column temperature was maintained at 25°C, and the sample compartment temperature was set to 10°C; and the detection wavelength was 214 nm.

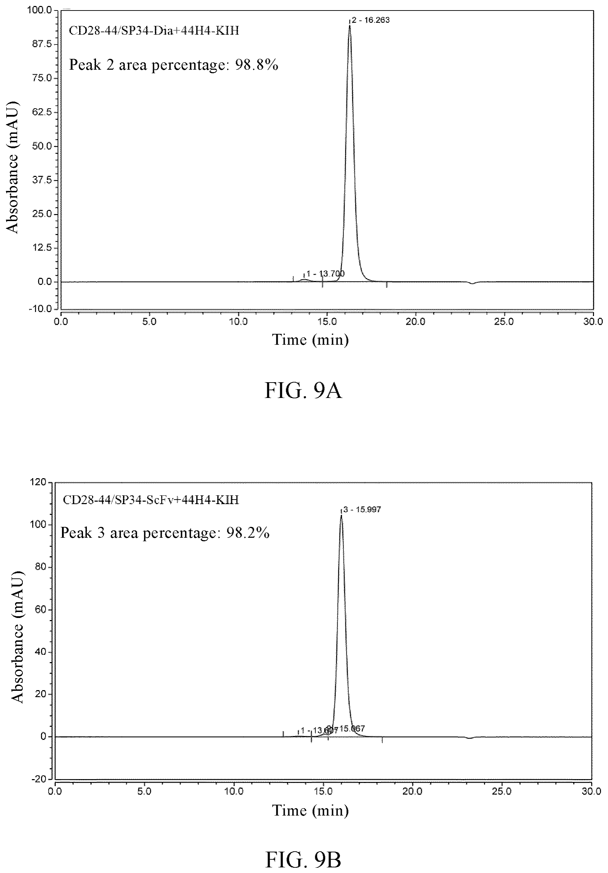

FIG. 9A shows the HPLC-SEC chromatogram of CD28-44/SP34-Dia+44H4-KIH, where the main peak with a retention time of 16.263 minutes accounts for 98.8%. FIG. 9B shows the HPLC-SEC chromatogram of CD28-44/SP34-ScFv+44H4-KIH, where the main peak with a retention time of 15.997 minutes accounts for 98.2%. The main peak percentages of these two trispecific antibodies are similar, indicating their comparable SEC purity.

The present invention employs CE-SDS (Capillary Electrophoresis-Sodium Dodecyl Sulfate) to analyze the content of degradation fragments or incompletely assembled molecules in a sample. CE is classified into non-reduced and reduced types. For the former, a reducing agent DTT is not required to disrupt intramolecular disulfide bonds during sample denaturation, and for the latter, the reducing agent DTT is required to disrupt intramolecular disulfide bonds during sample denaturation. The non-reduced and reduced CE-SDSes are denoted as NR-CE-SDS and R-CE-SDS, respectively. The capillary electrophoresis instrument used was ProteomeLab™ PA800 plus (Beckman Coulter), equipped with a UV 214 nm detector. The capillary was Bare Fused-Silica Capillary, with specifications of 30.7 cm $\times$ 50 $\mu$m and an effective length of 20.5 cm. Other related reagents were purchased from Beckman Coulter. The key instrument parameters were set as follows: the temperature for the capillary and sample chamber was $20 \pm 2$°C, and the separation voltage was 15 kV.

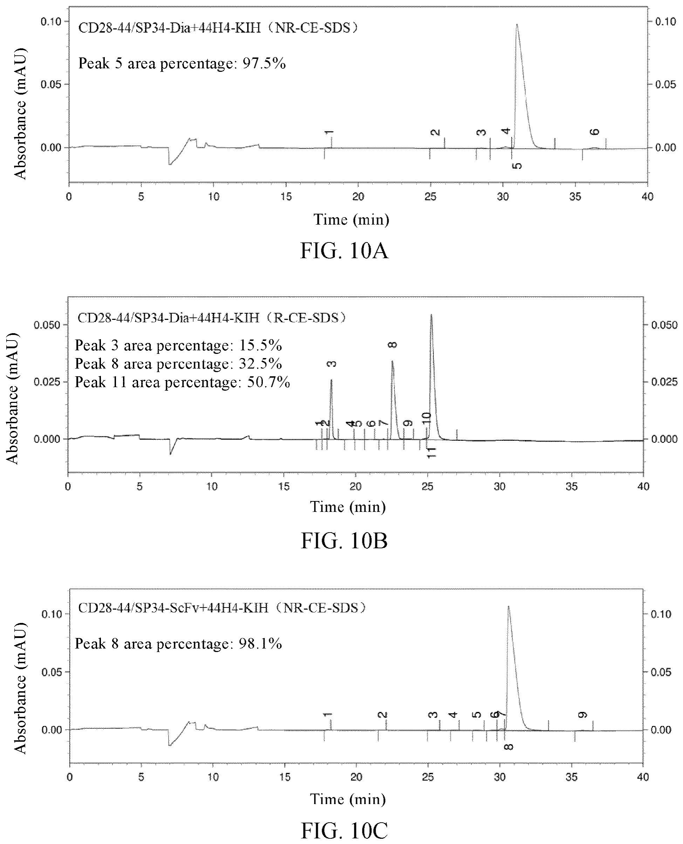

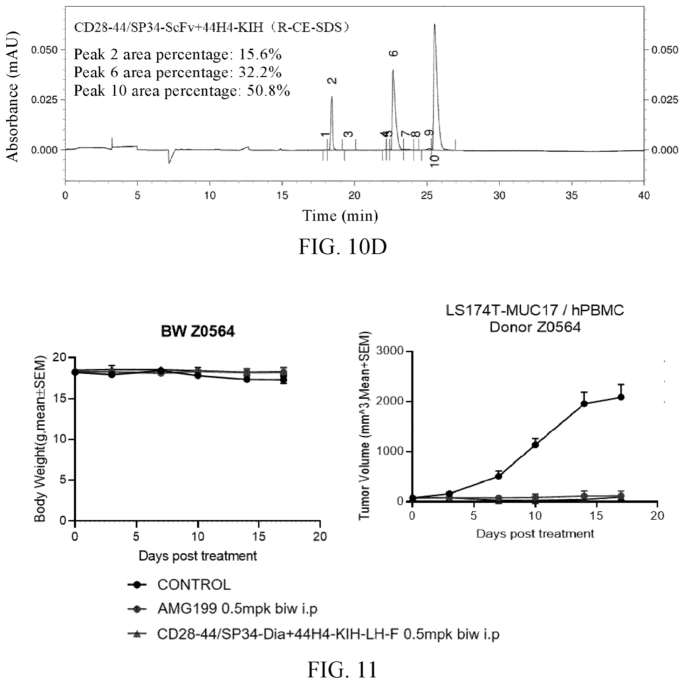

FIGs. 10A and 10B show the NR-CE-SDS and R-CE-SDS spectra of CD28-44/SP34-Dia+44H4-KIH, respectively, and FIGs. 10C and 10D show the NR-CE-SDS and R-CE-SDS spectra of CD28-44/SP34-ScFv+44H4-KIH, respectively. The main peak percentage of NR-CE-SDS for CD28-44/SP34-Dia+44H4-KIH is 97.5%, and that for CD28-44/SP34-ScFv+44H4-KIH is 98.1%. The main peaks 3, 8, and 11 (corresponding to the three polypeptide chains of the trispecific antibody in order of increasing molecular weight, respectively) of R-CE-SDS for CD28-44/SP34-Dia+44H4-KIH account for 15.5%, 32.5%, and 50.7%, respectively, with the combined peak area percentage totaling 98.7%. The main peaks 2, 6, and 10 (corresponding to the three polypeptide chains of the trispecific antibody in order of increasing molecular weight, respectively) of R-CE-SDS for CD28-44/SP34-ScFv+44H4-KIH account for 15.6%, 32.2%, and 50.8%, respectively, with the combined peak area percentage totaling 98.6%.

The results above show that both trispecific antibodies of the present invention exhibit SEC purity exceeding 98%, with CE-measured purity approaching or exceeding 98%, indicating their high purity and favorable physicochemical properties.

**Example 12. Evaluation of Animal Tumor Suppression Efficacy of Anti-MUC17*CD3*CD28 Trispecific Antibody**

Human peripheral blood mononuclear cells (hPBMCs) were used to reconstruct a humanized immune system in NOG (Vital River) mice, and a modified human colon cancer LS174T-MUC17 subcutaneous transplantation tumor model was established in these mice to evaluate the in vivo anti-tumor efficacy of the anti-MUC17*CD3*CD28 trispecific antibody. The method for preparing target cells is described as follows. The MUC17 gene was cloned into the pLVX-puro lentiviral vector (purchased from Clontech, Cat. No.: 632164), with the resulting expression vector designated as Muc17-plvx. Lentiviral packaging plasmids and the Muc17-plvx were co-transfected into HEK293 cells; a cell culture supernatant containing recombinant lentiviruses was collected to infect LS 174T cells; and after three days, pressurized screening was carried out with puromycin to obtain clones of cells transfected with the target genes. The final cell strains stably expressing MUC17 were designated as LS174T-MUC17. The specific implementation steps were as follows. 10 days before tumor cell seeding, the purchased PBMCs (Peripheral blood mononuclear cells, OriBiotech, Cat. No.: FPB004F-C) were thawed in vitro and resuspended with PBS, and the concentration of the PBMC suspension was adjusted to $2.5 \times 10^7$/mL. The mice were injected via the tail vein, with each mouse receiving 200 $\mu$L of PBMC cell suspension, equivalent to $5 \times 10^6$

PBMC cells per mouse. After 10 days, the modified human colon cancer LS174T-MUC17 cells cultured in vitro were collected, the concentration of the cell suspension was adjusted to $4 \times 10^7$/mL, and the cell suspension was mixed with matrigel at a 1:1 volume ratio. Under sterile conditions, 100 μL of cell suspension was inoculated subcutaneously into the right flank of each NOG mouse. The tumor cells to be inoculated formed solid tumors subcutaneously, with a volume of about 50-100 mm$^3$, and the mice were randomly grouped based on tumor volume. A blank control group was injected with saline only to serve as the control; a positive control antibody AMG199 group was administered at a dose of 0.5 mg/kg; an MUC17*CD3*CD28 group was administrated at a dose of 0.5 mg/kg. Subsequently, a pre-designed dosing regimen was administered twice a week for a total of 5 doses, with tumor volume measured twice weekly. Finally, the growth curves of tumors in each group over time were determined as shown in FIG. 11.

The results show that the anti-MUC17*CD3*CD28 trispecific antibody exhibits relatively higher tumor suppression efficacy than AMG199.

**Sequence Listing**

The amino acid sequence (SEQ ID NO: 1) of the heavy chain variable region of the anti-MUC17 murine monoclonal antibody 44H4 is as follows:

EVQLQQSGAELVKPGASVKLSFTASGFNIKDTYIHWVKQRPEQGLEWIGRIDPANGYTKYGPRFQGKATI
TADTASNAAYLQLSSLTSEDTAVYYCARNYGTSYPNAMDYWGQGTSVTVSS

The amino acid sequence (SEQ ID NO: 2) of the light chain variable region of the anti-MUC17 murine monoclonal antibody 44H4 is as follows:

DIVLTQSPASLAVSLGQRATISCRASESVETYGNSFMHWYQQKPGQPPKLLIYRASSLESGIPARFSGSGSR
TDFTLTITPVEADDVATYFCQQSNEDPYTFGGGTKLEIK

| | | |
|---|---|---|
| H-CDR1 of murine monoclonal antibody 44H4 | (SEQ ID NO. 3) | DTYIH |
| H-CDR2 of murine monoclonal antibody 44H4 | (SEQ ID NO. 4) | RIDPANGYTKYGPRFQG |
| H-CDR3 of murine monoclonal antibody 44H4 | (SEQ ID NO. 5) | NYGTSYPNAMDY |
| L-CDR1 of murine monoclonal antibody 44H4 | (SEQ ID NO. 6) | RASESVETYGNSFMH |
| L-CDR2 of murine monoclonal antibody 44H4 | (SEQ ID NO. 7) | RASSLES |
| L-CDR3 of murine monoclonal antibody 44H4 | (SEQ ID NO. 8) | QQSNEDPYT |

Amino acid sequence(SEQ ID NO: 9) of 44H4 humanized heavy chain variable region:

QVQLVQSGAEVKKPGASVKVSCKASGFNIKDTYIHWVRQAPGQGLEWMGRIDPANGYTKYGPRFQGRV
TITADTSTSTVYMELSSLRSEDTAVYYCARNYGTSYPNAMDYWGQGTLVTVSS

Amino acid sequence (SEQ ID NO: 10) of 44H4 humanized light chain variable region:

DIVLTQSPASLAVSPGQRATITCRASESVETYGNSFMHWYQQKPGQPPKLLIYRASSLESGVPARFSGSGS
GTDFTLTINPVEADDTANYYCQQSNEDPYTFGQGTKVEIK

Amino acid sequence (SEQ ID NO: 11) of human IgG1 heavy chain variable region:

ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNKA
LPAPIEKTISKAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK

Amino acid sequence of 44H4-Hu-HC (SEQ ID NO: 12):

QVQLVQSGAEVKKPGASVKVSCKASGFNIKDTYIHWVRQAPGQGLEWMGRIDPANGYTKYGPRFQGRV
TITADTSTSTVYMELSSLRSEDTAVYYCARNYGTSYPNAMDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

Amino acid sequence (SEQ ID NO: 13) of human Kappa light chain constant region:

RTVAAPSVFIFPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC

Amino acid sequence (SEQ ID NO: 14) of 44H4-Hu-LC:

DIVLTQSPASLAVSPGQRATITCRASESVETYGNSFMHWYQQKPGQPPKLLIYRASSLESGVPARFSGSGS
GTDFTLTINPVEADDTANYYCQQSNEDPYTFGQGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC

Amino acid sequence (SEQ ID NO: 15) of SP34 heavy chain variable region:

EVKLLESGGGLVQPKGSLKLSCAASGFTFNTYAMNWVRQAPGKGLEWVARIRSKYNNYATYYADSVKD
RFTISRDDSQSILYLQMNNLKTEDTAMYYCVRHGNFGNSYVSWFAYWGQGTLVTVSA

Amino acid sequence (SEQ ID NO: 16) of SP34 light chain variable region:

QAVVTQESALTTSPGETVTLTCRSSTGAVTTSNYANWVQEKPDHLFTGLIGGTNKRAPGVPARFSGSLIG
DKAALTITGAQTEDEAIYFCALWYSNLWVFGGGTKLTVL

| | | |
|---|---|---|
| H-CDR1 of SP34 antibody | (SEQ ID NO. 17) | TYAMN |
| H-CDR2 of SP34 antibody | (SEQ ID NO. 18) | RIRSKYNNYATYYADSVKD |
| H-CDR3 of SP34 antibody | (SEQ ID NO. 19) | HGNFGNSYVSWFAY |
| L-CDR1 of SP34 antibody | (SEQ ID NO. 20) | RSSTGAVTTSNYAN |
| L-CDR2 of SP34 antibody | (SEQ ID NO. 21) | GTNKRAP |
| L-CDR3 of SP34 antibody | (SEQ ID NO. 22) | ALWYSNLWV |

Amino acid sequence (SEQ ID NO: 23) of SP34 humanized heavy chain variable region:

EVQLVESGGGLVQPGGSLRLSCAASGFTFSTYAMNWVRQAPGKGLEWVSRIRSKYNNYATYYADSVKD
RFTISRDDSKNTLYLQMNSLRAEDTAVYYCARHDNFGNSYVSWFAYWGQGTLVTVSS

Amino acid sequence (SEQ ID NO: 24) of SP34 humanized light chain variable region:

QTVVTQEPSLTVSPGGTVTLTCGSSTGAVTTSNYPNWVQQKPGQAPRGLIGGTNKRAPGVPARFSGSLLG
GKAALTLSGAQPEDEAEYYCALWYSDLWVFGGGTKLTVL

Amino acid sequence (SEQ ID NO: 25) of SP34-Hu-HC:

EVQLVESGGGLVQPGGSLRLSCAASGFTFSTYAMNWVRQAPGKGLEWVSRIRSKYNNYATYYADSVKD
RFTISRDDSKNTLYLQMNSLRAEDTAVYYCARHDNFGNSYVSWFAYWGQGTLVTVSSASTKGPSVFPLA
PSSKSTSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPGK

Amino acid sequence (SEQ ID NO: 26) of Lambda light chain constant region:

GQPKAAPSVTLFPPSSEELQANKATLVCLISDFYPGAVTVAWKADSSPVKAGVETTTPSKQSNNKYAASS
YLSLTPEQWKSHRSYSCQVTHEGSTVEKTVAPTECS

Amino acid sequence (SEQ ID NO: 27) of SP34-Hu-LC:

QTVVTQEPSLTVSPGGTVTLTCGSSTGAVTTSNYPNWVQQKPGQAPRGLIGGTNKRAPGVPARFSGSLLG
GKAALTLSGAQPEDEAEYYCALWYSDLWVFGGGTKLTVLGQPKAAPSVTLFPPSSEELQANKATLVCLIS
DFYPGAVTVAWKADSSPVKAGVETTTPSKQSNNKYAASSYLSLTPEQWKSHRSYSCQVTHEGSTVEKTV
APTECS

Amino acid sequence (SEQ ID NO: 28) of Anti-CD28 heavy chain variable region:

QVQLVQSGAEVKKPGASVKVSCKASGYTFTSYYIHWVRQAPGQGLEWIGCIYPGNVNTNYNEKFKDRA
TLTVDTSISTAYMELSRLRSDDTAVYFCTRSHYGLDWNFDVWGQGTTVTVSS

Amino acid sequence (SEQ ID NO: 29) of Anti-CD28 light chain variable region:

DIQMTQSPSSLSASVGDRVTITCHASQNIYVWLNWYQQKPGKAPKLLIYKASNLHTGVPSRFSGSGSGTD
FTLTISSLQPEDFATYYCQQGQTYPYTFGGGTKVEIK

| H-CDR1 of Anti-CD28 antibody | (SEQ ID NO. 30) | SYYIH |
| H-CDR2 of Anti-CD28 antibody | (SEQ ID NO. 31) | CIYPGNVNTNYNEKFKD |
| H-CDR3 of Anti-CD28 antibody | (SEQ ID NO. 32) | SHYGLDWNFDV |
| L-CDR1 of Anti-CD28 antibody | (SEQ ID NO. 33) | HASQNIYVWLN |
| L-CDR2 of Anti-CD28 antibody | (SEQ ID NO. 34) | KASNLHT |
| L-CDR3 of Anti-CD28 antibody | (SEQ ID NO. 35) | QQGQTYPYT |

Amino acid sequence of Anti-CD28-Hu-HC (SEQ ID NO: 36):

QVQLVQSGAEVKKPGASVKVSCKASGYTFTSYYIHWVRQAPGQGLEWMGSIYPGNVNTNYNEKFKDRV
TLTVDTSISTAYMELSRLRSDDTAVYYCTRSHYGLDWNFDVWGQGTTVTVSSASTKGPSVFPLAPSSKST
SGGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGK

Amino acid sequence (SEQ ID NO: 37) of Anti-CD28-Hu-LC:

DIQMTQSPSSLSASVGDRVTITCHASQNIYVWLNWYQQKPGKAPKLLIYKASNLHTGVPSRFSGSGSGTD
FTLTISSLQPEDFATYYCQQGQTYPYTFGGGTKVEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC

Amino acid sequence (SEQ ID NO: 38) of CD28-44-SP34-Dia-IgG1-Knob-HC:

DIQMTQSPSSLSASVGDRVTITCHASQNIYVWLNWYQQKPGKAPKLLIYKASNLHTGVPSRFSGSGSGTD
FTLTISSLQPEDFATYYCQQGQTYPYTFGCGTKVEIKGGGGSEVQLVESGGGLVQPGGSLRLSCAASGFTF
STYAMNWVRQAPGKGLEWVSRIRSKYNNYATYYADSVKDRFTISRDDSKNTLYLQMNSLRAEDTAVYY
CARHDNFGNSYVSWFAYWGQGTLVTVSSGGGGSGGGGSGGGGSGGGGSQTVVTQEPSLTVSPGGTVTL
TCGSSTGAVTTSNYPNWVQQKPGQAPRGLIGGTNKRAPGVPARFSGSLLGGKAALTLSGAQPEDEAEYY
CALWYSDLWVFGGGTKLTVLGGGGSQVQLVQSGAEVKKPGASVKVSCKASGYTFTSYYIHWVRQAPG
QCLEWMGSIYPGNVNTNYNEKFKDRVTLTVDTSISTAYMELSRLRSDDTAVYYCTRSHYGLDWNFDVW
GQGTTVTVSSGGGGSGGGGSGGGGSDKTHTCPPCPAPEAAGAPSVFLFPPKPKDTLMISRTPEVTCVVVD
VSHEDPEVKFNWYVDGVEVHNAKTKPREEQYNSTYRVVSVLTVLHQDWLNGKEYKCKVSNKALPAPIE
KTISKAKGQPREPQVYTLPPCRDELTKNQVSLWCLVKGFYPSDIAVEWESNGQPENNYKTTPPVLDSDGS
FFLYSKLTVDKSRWQQGNVFSCSVMHEALHNHYTQKSLSLSPGK

Nucleotide sequence (SEQ ID NO: 39) of CD28-44-SP34-Dia-IgG1-Knob-HC:

GACATCCAGATGACCCAGAGTCCCAGCAGCCTGTCTGCTTCTGTGGGAGATAGAGTGACAATTACAT
GTCATGCTTCTCAGAATATCTACGTGTGGCTGAACTGGTACCAGCAGAAACCTGGAAAGGCTCCTAA

GCTGCTGATCTACAAGGCTTCTAACCTGCACACAGGCGTGCCTTCTAGATTTTCTGGATCTGGCTCCG
GAACAGATTTTACTCTGACCATCTCTAGCCTGCAGCCAGAAGATTTTGCCACCTATTATTGTCAGCAG
GGACAGACCTACCCTTACACATTTGGCTGCGGCACAAAGGTGGAGATCAAGGGTGGAGGCGGTAGC
GAGGTGCAGCTGGTGGAGTCTGGCGGCGGCCTGGTGCAGCCTGGCGGCTCTCTGAGACTGTCTTGTG
CTGCTTCTGGCTTTACCTTTTCTACCTATGCTATGAATTGGGTGAGACAGGCTCCTGGCAAGGGCCTG
GAGTGGGTGTCTAGAATCAGATCTAAGTATAATAATTACGCTACCTATTATGCTGATTCTGTGAAGGA
CAGATTCACAATCTCTAGAGATGATTCTAAGAATACCCTGTATCTGCAGATGAACTCTCTGAGAGCTG
AGGATACCGCTGTGTATTACTGTGCTAGACATGACAATTTTGGCAACTCTTATGTGTCTTGGTTTGCTT
ATTGGGGACAGGGAACACTGGTGACAGTGAGCTCTGGTGGAGGCGGTTCAGGCGGCGGAGGCAGCG
GAGGTGGCGGGAGTGGAGGGGGTGGCTCTCAGACCGTGGTGACACAGGAGCCCAGCCTGACCGTGA
GCCCCGGCGGCACCGTGACCCTGACCTGCGGCTCCTCCACAGGCGCCGTGACCACCTCCAACTACCC
CAACTGGGTGCAGCAGAAGCCCGGCCAGGCCCCAAGGGGCCTGATCGGCGGCACCAATAAGAGGGC
CCCCGGCGTGCCCGCTAGATTCTCTGGCTCTCTTCTGGGAGGAAAGGCTGCTCTGACACTGTCTGGAG
CTCAGCCCGAGGATGAGGCTGAATACTATTGTGCTCTGTGGTATTCTGATCTGTGGGTGTTTGGCGGT
GGAACTAAACTTACAGTTCTGGGCGGGGGCGGTAGCCAGGTGCAGCTGGTCCAGAGCGGAGCCGAG
GTGAAGAAGCCCGGCGCCAGCGTGAAGGTGAGCTGCAAGGCCAGCGGCTACACTTTTACCTCTTACT
ACATTCACTGGGTGAGACAGGCTCCCGGCCAGTGCCTGGAGTGGATGGGATCCATCTATCCTGGAAA
TGTGAACACCAACTATAATGAGAAGTTTAAAGATAGAGTGACCCTGACCGTGGATACATCTATCTCT
ACCGCTTATATGGAGCTGTCTAGACTGAGATCCGATGACACAGCTGTGTATTATTGTACCAGATCTCA
TTACGGACTGGATTGGAATTTTGATGTGTGGGGCCAGGGCACCACAGTGACCGTGTCTTCTGGTGGG
GGTGGTTCAGGGGGTGGTGGAAGTGGCGGTGGAGGGAGTGACAAGACCCACACATGTCCCCCCTGT
CCCGCTCCTGAAGCTGCAGGAGCCCCTTCCGTGTTCCTGTTCCCCCCTAAGCCCAAGGACACCCTGAT
GATTTCCAGGACACCCGAGGTGACCTGTGTGGTGGTGGACGTCAGCCACGAGGACCCCGAGGTGAA
ATTCAACTGGTACGTCGATGGCGTGGAGGTGCACAACGCTAAGACCAAGCCCAGGGAGGAGCAGTA
CAATTCCACCTACAGGGTGGTGTCCGTGCTGACCGTCCTCCATCAGGACTGGCTGAACGGCAAAGAG
TATAAGTGCAAGGTGAGCAACAAGGCCCTCCCTGCTCCCATCGAGAAGACCATCAGCAAAGCCAAG
GGCCAGCCCAGGGAACCTCAAGTCTATACCCTGCCTCCCTGCAGGGATGAGCTGACCAAGAACCAAG
TGAGCCTCTGGTGCCTCGTCAAGGGCTTCTATCCTTCCGATATTGCCGTCGAGTGGGAGTCCAACGGA
CAGCCCGAGAACAACTACAAGACAACACCCCCGTGCTCGATTCCGATGGCAGCTTCTTCCTGTACT
CCAAGCTGACCGTGGACAAGTCCAGATGGCAACAAGGCAACGTCTTCAGTTGCAGCGTCATGCATGA
GGCCCTCCACAACCACTACACCCAGAAGAGCCTCTCCCTGAGCCCTGGAAAG

Amino acid sequence (SEQ ID NO: 40) of 44H4-IgG1-Hole-HC:

QVQLVQSGAEVKKPGASVKVSCKASGFNIKDTYIHWVRQAPGQGLEWMGRIDPANGYTKYGPRFQGRV
TITADTSTSTVYMELSSLRSEDTAVYYCARNYGTSYPNAMDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPEAAGAPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQP
REPQVCTLPPSRDELTKNQVSLSCAVKGFYPSDIAVEWESNGQPENNYKTTPPVLDSDGSFFLVSKLTVDK
SRWQQGNVFSCSVMHEALHNRFTQKSLSLSPGK

Nucleotide sequence (SEQ ID NO: 41) of 44H4-IgG1-Hole-HC:

CAGGTGCAGCTGGTGCAGTCTGGCGCTGAGGTGAAGAAGCCTGGCGCTTCTGTGAAGGTGTCTTGTA
AGGCTTCTGGCTTTAATATCAAGGATACATATATCCATTGGGTGAGACAGGCCCCTGGCCAGGGCCT
GGAGTGGATGGGCAGAATCGATCCAGCTAATGGCTACACCAAGTATGGCCCTAGATTTCAGGGAAG
AGTGACCATCACAGCTGATACATCTACCTCCACCGTGTATATGGAGCTGTCCTCTGAGATCTGAGG
ATACAGCTGTGTATTATTGTGCTAGAAATTATGGCACATCTTACCCTAATGCTATGGATTACTGGGGA
CAGGGAACACTGGTGACAGTGAGCTCTGCTTCCACCAAAGGACCCAGCGTGTTCCTCTGGCCCCTTC
CAGCAAGAGCACCAGCGGAGGCACAGCTGCCCTGGGATGCCTGGTGAAGGACTATTTCCCTGAGCCC
GTGACAGTCAGCTGGAACTCCGGCGCTCTGACCTCCGGCGTCCACACCTTTCCTGCCGTGCTGCAGAG
CAGCGGCCTGTACAGCCTGTCCAGCGTGGTGACCGTGCCTAGCTCCAGCCTGGGCACCCAGACCTAC
ATCTGCAACGTGAACCACAAGCCCTCCAACACCAAGGTGGACAAGAAGGTGGAGCCTAAGAGCTGT
GACAAGACCCACACATGCCCTCCTTGTCCTGCTCCTGAAGCCGCCGGCGCACCTTCCGTGTTCCTGTT
CCCCCCTAAGCCCAAGGATACCCTCATGATTTCCAGGACCCCCGAAGTGACCTGCGTGGTGGTCGAT
GTGAGCCACGAAGACCCTGAAGTGAAGTTCAACTGGTACGTGGACGGCGTCGAGGTCCACAACGCC
AAAACCAAGCCCAGGGAGGAGCAGTACAATAGCACCTACCGGGTGGTGAGCGTGCTGACCGTGCTG
CACCAGGACTGGCTGAACGGCAAGGAGTACAAGTGTAAGGTCAGCAACAAGGCCCTCCCTGCCCCC
ATCGAGAAGACCATTTCCAAGGCTAAGGGACAGCCTCGGGAACCCCAAGTGTGCACCCTGCCTCCTA
GCAGGGATGAGCTGACCAAGAACCAGGTGAGCCTGAGCTGTGCTGTGAAGGGCTTCTACCCTTCCGA
CATCGCTGTCGAGTGGGAGAGCAACGGCCAGCCCGAGAATAACTACAAGACAACCCCCCCGTGCTC
GACAGCGATGGCAGCTTCTTTCTGGTGTCCAAGCTCACCGTGGACAAGTCCAGGTGGCAGCAGGGCA
ACGTCTTCTCCTGCTCCGTCATGCACGAGGCCCTGCACAACCGCTTCACACAGAAGTCCCTGTCCCTG
TCCCCTGGAAAG

Amino acid sequence (SEQ ID NO: 42) of CD28-44-SP34-2ScFv-IgG1-Knob-HC:

DIQMTQSPSSLSASVGDRVTITCHASQNIYVWLNWYQQKPGKAPKLLIYKASNLHTGVPSRFSGSGSGTD
FTLTISSLQPEDFATYYCQQGQTYPYTFGCGTKVEIKGGGGSGGGGSGGGGSGGGGSQVQLVQSGAEVK
KPGASVKVSCKASGYTFTSYYIHWVRQAPGQCLEWMGSIYPGNVNTNYNEKFKDRVTLTVDTSISTAYM
ELSRLRSDDTAVYYCTRSHYGLDWNFDVWGQGTTVTVSSGGGSGGGGSQTVVTQEPSLTVSPGGTVTLTC
GSSTGAVTTSNYPNWVQQKPGQAPRGLIGGTNKRAPGVPARFSGSLLGGKAALTLSGAQPEDEAEYYCA
LWYSDLWVFGGGTKLTVLGGGGSGGGGSGGGGSGGGGSEVQLVESGGGLVQPGGSLRLSCAASGFTFS
TYAMNWVRQAPGKGLEWVSRIRSKYNNYATYYADSVKDRFTISRDDSKNTLYLQMNSLRAEDTAVYYC
ARHDNFGNSYVSWFAYWGQGTLVTVSSGGGSGGGGSDKTHTCPPCPAPEAAGAPSVFLFPPKPKDTLMIS
RTPEVTCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPCRDELTKNQVSLWCLVKGFYPSDIAVEWESNGQPENNY
KTTPPVLDSDGSFFLYSKLTVDKSRWQQGNVFSCSVMHEALHNHYTQKSLSLSPGK

Nucleotide sequence (SEQ ID NO: 43) of CD28-44-SP34-2ScFv-IgG1-Knob-HC:

GACATCCAGATGACCCAGAGTCCCAGCAGCCTGTCTGCTTCTGTGGGAGATAGAGTGACAATTACAT
GTCATGCTTCTCAGAATATCTACGTGTGGCTGAACTGGTACCAGCAGAAACCTGGAAAGGCTCCTAA
GCTGCTGATCTACAAGGCTTCTAACCTGCACACAGGCGTGCCTTCTAGATTTTCTGGATCTGGCTCCG
GAACAGATTTTACTCTGACCATCTCTAGCCTGCAGCCAGAAGATTTTGCCACCTATTATTGTCAGCAG
GGACAGACCTACCCTTACACATTTGGCTGCGGCACAAAGGTGGAGATCAAGGGTGGAGGCGGTTCA
GGCGGCGGAGGCAGCGGAGGTGGCGGGAGTGGAGGGGGTGGCTCTCAGGTGCAGCTGGTCCAGAGC
GGAGCCGAGGTGAAGAAGCCCGGCGCCAGCGTGAAGGTGAGCTGCAAGGCCAGCGGCTACACTTTT
ACCTCTTACTACATTCACTGGGTGAGACAGGCTCCCGGCCAGTGCCTGGAGTGGATGGGATCTATCT
ATCCTGGAAATGTGAACACCAACTATAATGAGAAGTTTAAAGATAGAGTGACCCTGACCGTGGATAC
ATCTATCTCTACCGCTTATATGGAGCTGTCTAGACTGAGATCCGATGACACAGCTGTGTATTATTGTA
CCAGATCTCATTACGGACTGGATTGGAATTTTGATGTGTGGGGCCAGGGCACCACAGTGACCGTGTC
TTCTGGGGGCGGTAGCGGGGGCGGGTCTCAGACCGTGGTGACACAGGAGCCCAGCCTGACCGTGAG
CCCCGGCGGCACCGTGACCCTGACCTGCGGCTCCTCCACCGGCGCCGTGACCACCTCCAACTACCCC
AACTGGGTGCAGCAGAAGCCCGGCCAGGCCCCAAGGGGCCTGATCGGCGGCACCAATAAGAGGGCC
CCCGGCGTGCCCGCTAGATTCTCTGGCTCTCTTCTGGGAGGAAAGGCTGCTCTGACACTGTCTGGAGC
TCAGCCCGAGGATGAGGCTGAATACTATTGTGCTCTGTGGTATTCTGATCTGTGGGTGTTTGGCGGTG
GAACTAAACTTACAGTTCTGGGTGGGGGTGGTTCAGGGGGTGGTGGAAGTGGCGGTGGAGGGAGTG
GTGGAGGCGGTAGCGAGGTGCAGCTGGTGGAGTCTGGCGGCGGCCTGGTGCAGCCTGGCGGCTCTCT
GAGACTGTCTTGTGCTGCTTCTGGCTTTACCTTTTCTACCTATGCTATGAATTGGGTGAGACAGGCTCC
TGGCAAGGGCCTGGAGTGGGTGTCTAGAATCAGATCTAAGTATAATAATTACGCTACCTATTATGCT
GATTCTGTGAAGGACAGATTCACAATCTCTAGAGATGATTCTAAGAATACCCTGTATCTGCAGATGA
ACTCTCTGAGAGCTGAGGATACCGCTGTGTATTACTGTGCTAGACATGACAATTTTGGCAACTCTTAT
GTGTCTTGGTTTGCTTATTGGGGACAGGGAACACTGGTGACAGTGAGCTCTGGCGGGGGATCAGGAG
GTGGCAGTGACAAGACCCACACATGTCCCCCCTGTCCCGCTCCTGAAGCTGCAGGAGCCCCTTCCGT
GTTCCTGTTCCCCCCTAAGCCCAAGGACACCCTGATGATTTCCAGGACACCCGAGGTGACCTGTGTGG
TGGTGGACGTCAGCCACGAGGACCCCGAGGTGAAATTCAACTGGTACGTCGATGGCGTGGAGGTGC
ACAACGCTAAGACCAAGCCCAGGGAGGAGCAGTACAATTCCACCTACAGGGTGGTGTCCGTGCTGA
CCGTCCTCCATCAGGACTGGCTGAACGGCAAAGAGTATAAGTGCAAGGTGAGCAACAAGGCCCTCCC
TGCTCCCATCGAGAAGACCATCAGCAAAGCCAAGGGCCAGCCCAGGGAACCTCAAGTCTATACCCTG
CCTCCCTGCAGGGATGAGCTGACCAAGAACCAAGTGAGCCTCTGGTGCCTCGTCAAGGGCTTCTATC
CTTCCGATATTGCCGTCGAGTGGGAGTCCAACGGACAGCCCGAGAACAACTACAAGACAACACCCCC
CGTGCTCGATTCCGATGGCAGCTTCTTCCTGTACTCCAAGCTGACCGTGGACAAGTCCAGATGGCAAC
AAGGCAACGTCTTCAGTTGCAGCGTCATGCATGAGGCCCTCCACAACCACTACACCCAGAAGAGCCT
CTCCCTGAGCCCTGGAAAG

Amino acid sequence (SEQ ID NO: 44) of IgG1-Knob-HC:

DKTHTCPPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPCRDELT
KNQVSLWCLVKGFYPSDIAVEWESNGQPENNYKTTPPVLDSDGSFFLYSKLTVDKSRWQQGNVFSCSVM
HEALHNHYTQKSLSLSPGK

Amino acid sequence (SEQ ID NO: 45) of IgG1-Hole-HC:

DKTHTCPPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVCTLPPSRDELT
KNQVSLSCAVKGFYPSDIAVEWESNGQPENNYKTTPPVLDSDGSFFLVSKLTVDKSRWQQGNVFSCSVM
HEALHNRFTQKSLSLSPGK

Amino acid sequence (SEQ ID NO: 46) of MUC17-His:

RTTTCFGDGCQNTASRCKNGGTWDGLKCQCPNLYYGELCEEVVSSIDIGPPETISAQMELTVTVTSVKFTE
ELKNHSSQEFQEFKQTFTEQMNIVYSGIPEYVGVNITKLRLGSVVVEHDVLLRTKYTPEYKTVLDNATEV
VKEKITKVTTQQIMINDICSDMMCFNTTGTQVQNITVTQYDPEEDCRKMAKEYGDYFVVEYRDQKPYCIS
PCEPGFSVSKNCNLGKCQMSLSGPQCLCVTTETHWYSGETCNQGTQKSLGGGGSHHHHHH

Amino acid sequence (SEQ ID NO: 47) of CD28-His:

NKILVKQSPMLVAYDNAVNLSCKYSYNLFSREFRASLHKGLDSAVEVCVVYGNYSQQLQVYSKTGFNC
DGKLGNESVTFYLQNLYVNQTDIYFCKIEVMYPPPYLDNEKSNGTIIHVKGKHLCPSPLFPGPSKPGGGGS
HHHHHH

Amino acid sequence (SEQ ID: 48) of CD28-CD3-CODV-IgG1-Knob-HC:

QVQLVQSGAEVVKPGASVKVSCKASGYTFTSYYIHWVRQAPGQGLEWIGSIYPGNVNTNYAQKFQGRA
TLTVDTSISTAYMELSRLRSDDTAVYYCTRSHYGLDWNFDVWGKGTTVTVSSSQVQLVESGGGVVQPGR
SLRLSCAASGFTFTKAWMHWVRQAPGKQLEWVAQIKDKSNSYATYYADSVKGRFTISRDDSKNTLYLQ
MNSLRAEDTAVYYCRGVYYALSPFDYWGQGTLVTVSSRTASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKKVEPK
SCDKTHTCPPCPAPEAAGAPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQVYTLPPCRDEL
TKNQVSLWCLVKGFYPSDIAVEWESNGQPENNYKTTPPVLDSDGSFFLYSKLTVDKSRWQQGNVFSCSV
MHEALHNHYTQKSLSLSPGK

Amino acid sequence (SEQ ID NO: 49) of CD3-CD28-CODV-LC:

DIVMTQTPLSLSVTPGQPASISCKSSQSLVHNNANTYLSWYLQKPGQSPQSLIYKVSNRFSGVPDRFSGSGS
GTDFTLKISRVEAEDVGVYYCGQGTQYPFTFGSGTKVEIKGQPKAAPDIQMTQSPSSLSASVGDRVTITCQ
ASQNIYVWLNWYQQKPGKAPKLLIYKASNLHTGVPSRFSGSGSGTDFTLTISSLQPEDIATYYCQQGQTYP
YTFGQGTKLEIKTKGPSRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

Amino acid sequence (SEQ ID NO: 50) of modified anti-CD28 heavy chain variable region:

QVQLVQSGAEVKKPGASVKVSCKASGYTFTSYYIHWVRQAPGQGLEWMGSIYPGNVNTNYNEKFKDRV
TLTVDTSISTAYMELSRLRSDDTAVYYCTRSHYGLDWNFDVWGQGTTVTVSS

| | |
|---|---|
| Humanized SP34 antibody H-CDR3 (SEQ ID NO. 51) | HDNFGNSYVSWFAYHDNFGNSYVSWFAY |
| Humanized SP34 antibody L-CDR3 (SEQ ID NO. 52) | ALWYSDLWV |
| Humanized Anti-CD28 antibody H-CDR2 (SEQ ID NO. 53) | SIYPGNVNTNYNEKFKD |

All publications mentioned in the present invention are incorporated in the present application by reference to the same extent as if each individual publication was individually indicated to be incorporated by reference. Furthermore, it should be understood that, after reading the above teachings of the present invention, those skilled in the art can make various changes or modifications to the present invention, and these equivalent forms shall also fall within the scope defined by the claims attached to the present application.

**Claims**

1. A multispecific T-cell engager, comprising:

   a first targeting domain D1, wherein the D1 binds to a target protein selected from the group consisting of: CD3, CD28, CD40, CD137;
   an optional second targeting domain D2, wherein the D2 binds to a target protein selected from the group consisting of: CD3, CD28, CD40, CD137; and
   a third targeting domain D3, wherein the D3 comprises one or more MUC17 antigen-binding domains.

2. The multispecific T-cell engager according to claim 1, **characterized in that** said D1, D2 or D3 is each independently selected from: single-domain antibody (sdAb), Fab, Fab', F(ab')$_2$, TriFab, Fv fragment, fragment variable Fv heterodimer, single-chain Fv (scFv) fragment, diabody, bispecific T-cell engager (BiTE), or single-domain fragment, preferably single-chain Fv (scFv), Fv fragment, or Fab fragment.

3. The multispecific T-cell engager according to claim 1, **characterized in that** said MUC17 antigen-binding domain comprises a third heavy chain variable region and a third light chain variable region, wherein the third heavy chain variable region comprises three heavy chain variable regions CDRs as follows:

H-CDR1 having an amino acid sequence as set forth in SEQ ID NO: 3;
H-CDR2 having an amino acid sequence as set forth in SEQ ID NO: 4; and
H-CDR3 having an amino acid sequence as set forth in SEQ ID NO: 5; and
the third light chain variable region comprises three light chain variable regions CDRs as follows:

L-CDR1 having an amino acid sequence as set forth in SEQ ID NO: 6;
L-CDR2 having an amino acid sequence as set forth in SEQ ID NO: 7; and
L-CDR3 having an amino acid sequence as set forth in SEQ ID NO: 8.

4. The multispecific T-cell engager according to claim 1, **characterized in that** said multispecific T-cell engager further comprises an Fc fragment, preferably an Fc fragment derived from IgG1 or IgG4.

5. The multispecific T-cell engager according to claim 1, **characterized in that** said Fc fragment derived from IgG1 comprises mutations selected from the group consisting of:

N297A;
L234F/L235E/P331S;
Y349C/K370E/K409D/K439E;
S354C/D356K/E357K/D399K;
S354C/T366W;
S354C/T366W/H435R/Y436F;
Y349C/T366S/L368A/Y407V;
L234F/L235E/P331S/Y349C/K370E/K409D/K439E;
L234F/L235E/P331S/S354C/D356K/E357K/D399K;
L234F/L235E/P331S/S354C/T366W;
L234F/L235E/P331S/Y349C/T366S/L368A/Y407V;
N297A/Y349C/K370E/K409D/K439E;
N297A/S354C/D356K/E357K/D399K;
N297A/S354C/T366W;
N297A/Y349C/T366S/L368A/Y407V; and/or
S267E; S267E/G236D; S267E/S239D; S267E/L328F.

6. The multispecific T-cell engager according to claim 1, **characterized in that** said multispecific T-cell engager is a trispecific antibody comprising the following structure from N-terminus to C-terminus:

(a) a first chain: VL$_1$-L1-VH$_2$-L2-VL$_2$-L3-VH$_1$-L4-Fc1;
(b) a second chain: VH$_3$-CH1-Fc2; and
(c) a third chain: VL$_3$-CL;

or the trispecific antibody comprises the following structure from N-terminus to C-terminus:

(a) a first chain: VL$_1$-L1-VH$_1$-L2-VL$_2$-L3-VH$_2$-L4-Fc1;
(b) a second chain: VH$_3$-CH1-Fc2; and
(c) a third chain: VL$_3$-CL;

wherein,

VH$_1$ is a first heavy chain variable region, and VL$_1$ is a first light chain variable region;
VH$_2$ is a second heavy chain variable region, and VL$_2$ is a second light chain variable region;
VH$_3$ is a third heavy chain variable region, and VL$_3$ is a third light chain variable region;
CL is a light chain constant region, and CH1 is a CH1 domain;
L1, L2, L3 and L4 are each independently none, a bond or a linker;
Fc1 or Fc2 is independently an Fc element; and
"-" represents a peptide bond.

7. A polynucleotide encoding the multispecific T-cell engager according to any one of claims 1 to 6.

8. A vector comprising the polynucleotide according to claim 7.

9. A host cell comprising the vector according to claim 8 or having a genome incorporating the polynucleotide according to claim 7.

10. A method for preparing the multispecific T-cell engager according to any one of claims 1 to 6, comprising the steps of:

(i) culturing the host cell according to claim 9 under suitable conditions to obtain a mixture comprising the multispecific T-cell engager according to any one of claims 1 to 6;
(ii) purifying and/or separating the mixture obtained in step (i) to obtain the multispecific T-cell engager according to any one of claims 1 to 6.

11. A pharmaceutical composition, comprising:

(I) the multispecific T-cell engager according to any one of claims 1 to 6; and
(II) a pharmaceutically acceptable carrier.

12. An immunoconjugate, comprising:

(a) the multispecific T-cell engager according to any one of claims 1 to 6; and
(b) a conjugated moiety selected from the group consisting of: a detectable marker, a medicament, a toxin, a cytokine, a radionuclide, an enzyme, or combinations thereof.

13. Use of the multispecific T-cell engager according to any one of claims 1 to 6, the pharmaceutical composition according to claim 11, or the immunoconjugate according to claim 12 in preparation of: (a) a detection reagent or kit; and/or (b) a medicament for preventing and/or treating a cancer/tumor.

14. The use according to claim 13, **characterized in that** said cancer/tumor is MUC17-associated cancer/tumor.

15. The use according to claim 13, **characterized in that** said cancer/tumor comprises solid tumors and hematological malignancies.

16. The use according to claim 13, **characterized in that** said cancer/tumor is selected from the group consisting of: gastric cancer, gastrointestinal cancer, colorectal cancer, esophageal cancer, gastroesophageal cancer, pancreatic cancer, lung cancer, breast cancer, liver cancer, cervical cancer, thyroid cancer, uterine cancer, prostate cancer, or combinations thereof.

17. A kit, comprising the multispecific T-cell engager according to any one of claims 1 to 6, the polynucleotide according to claim 7, the vector according to claim 8, the host cell according to claim 9, the pharmaceutical composition according to claim 11, or the immunoconjugate according to claim 12.

FIG. 1A

FIG. 1B

MUC17-His

FIG. 2A

CD3-Epsilon

FIG. 2B

**CD28-His**

FIG. 2C

**LS 174T**

FIG. 3A

FIG. 3B

FIG. 4A

**LS174T-Luciferase**

FIG. 4B

**CD3-Epsilon**

FIG. 5A

**CD28-His**

FIG. 5B

**LS174T-Luciferase**

FIG. 6A

**CD3 T**

FIG. 6B

**LS174T-Luciferase**

FIG. 7A

CD3 T

FIG. 7B

PBMC

FIG. 8A

FIG. 8B

FIG. 8C

FIG. 9A

FIG. 9B

FIG. 10A

FIG. 10B

FIG. 10C

FIG. 10D

FIG. 11

## INTERNATIONAL SEARCH REPORT

| | |
|---|---|
| International application No. | **PCT/CN2024/081748** |

### A. CLASSIFICATION OF SUBJECT MATTER

C07K 16/30(2006.01)i; C12N 15/13(2006.01)i; C07K 16/18(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

IPC: C07K,C12N,A61K, A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, PubMed, ISI Web of Knowledge, CNKI, GenBank, 中国专利生物序列数据库, China Patents Biological Sequence Database: 多特异性抗体, 三特异性抗体, multi-specific antibody, tri-specific antibody, T细胞衔接子, T-cell engagers, CD3, CD28, CD40, CD137, MUC17, 黏蛋白17, 癌症, 肿瘤, tumor, cancer, SEQ ID Nos: 1-10

### C. DOCUMENTS CONSIDERED TO BE RELEVANT

| Category* | Citation of document, with indication, where appropriate, of the relevant passages | Relevant to claim No. |
|---|---|---|
| X | CN 111630067 A (AMGEN INC. et al.) 04 September 2020 (2020-09-04) claims 1-40, and description, paragraph 0247 | 1-2, 4-17 |
| Y | CN 111630067 A (AMGEN INC. et al.) 04 September 2020 (2020-09-04) claims 1-40, and description, paragraph 0247 | 3, 7-17 |
| A | CN 110799540 A (SYSTIMMUNE, INC. et al.) 14 February 2020 (2020-02-14) claims 1-26 | 1-17 |
| A | CN 101687924 A (FORERUNNER PHARMA RESEARCH CO., LTD.) 31 March 2010 (2010-03-31) claims 1-12 | 1-17 |
| A | CN 111836832 A (NANJING LEGEND BIOTECH CO., LTD.) 27 October 2020 (2020-10-27) claims 1-59 | 1-17 |
| A | CN 115427454 A (AMGEN INC.) 02 December 2022 (2022-12-02) claims 1-57 | 1-17 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **13 June 2024** | **20 June 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 682 172 A1**

## INTERNATIONAL SEARCH REPORT

International application No.

**PCT/CN2024/081748**

### C. DOCUMENTS CONSIDERED TO BE RELEVANT

| Category* | Citation of document, with indication, where appropriate, of the relevant passages | Relevant to claim No. |
|---|---|---|
| A | YANG, Bing et al. "Mucin 17 inhibits the progression of human gastric cancer by limiting inflammatory responses through a MYH9-p53-RhoA regulatory feedback loop" *Journal of Experimental & Clinical Cancer Research*, Vol. 38, 01 July 2019 (2019-07-01), article 283, pages 1-13 | 1-17 |
| PY | CN 116284408 A (SUNSHINE GUOJIAN PHARMACEUTICAL （SHANGHAI） CO., LTD.) 23 June 2023 (2023-06-23) claims 1-18 | 3, 7-17 |

Form PCT/ISA/210 (second sheet) (July 2022)

## INTERNATIONAL SEARCH REPORT

| International application No. |
| --- |
| **PCT/CN2024/081748** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2024/081748**

| Patent document cited in search report | | | Publication date (day/month/year) | Patent family member(s) | | | Publication date (day/month/year) |
|---|---|---|---|---|---|---|---|
| CN | 111630067 | A | 04 September 2020 | UY | 38041 | A | 28 June 2019 |
| | | | | JP | 2023134497 | A | 27 September 2023 |
| | | | | IL | 275492 | A | 31 August 2020 |
| | | | | WO | 2019133961 | A1 | 04 July 2019 |
| | | | | PH | 12020550841 | A1 | 17 May 2021 |
| | | | | JP | 2021508465 | A | 11 March 2021 |
| | | | | MA | 51417 | A | 04 November 2020 |
| | | | | CL | 2020001732 | A1 | 12 February 2021 |
| | | | | MX | 2020006871 | A | 24 August 2020 |
| | | | | BR | 112020013018 | A2 | 17 February 2021 |
| | | | | SG | 11202005274 | VA | 29 July 2020 |
| | | | | CA | 3087151 | A1 | 04 July 2019 |
| | | | | EP | 3732200 | A1 | 04 November 2020 |
| | | | | AU | 2018394255 | A1 | 11 June 2020 |
| | | | | CR | 20200325 | A | 09 November 2020 |
| | | | | KR | 20200105493 | A | 07 September 2020 |
| | | | | TW | 201940518 | A | 16 October 2019 |
| | | | | US | 2021130465 | A1 | 06 May 2021 |
| | | | | JOP | 20200158 | A1 | 30 October 2022 |
| | | | | CO | 2020009034 | A2 | 31 July 2020 |
| | | | | PE | 20201340 | A1 | 25 November 2020 |
| CN | 110799540 | A | 14 February 2020 | US | 2020157224 | A1 | 21 May 2020 |
| | | | | IL | 271260 | A | 30 January 2020 |
| | | | | WO | 2019005640 | A2 | 03 January 2019 |
| | | | | WO | 2019005640 | A3 | 07 February 2019 |
| | | | | KR | 20200092302 | A | 03 August 2020 |
| | | | | EP | 3645048 | A2 | 06 May 2020 |
| | | | | EP | 3645048 | A4 | 16 June 2021 |
| | | | | RU | 2020102663 | A | 27 July 2021 |
| | | | | RU | 2020102663 | A3 | 06 April 2022 |
| | | | | JP | 2020530306 | A | 22 October 2020 |
| | | | | JP | 7474193 | B2 | 24 April 2024 |
| | | | | SG | 11201912865 | VA | 30 January 2020 |
| | | | | AU | 2018295119 | A1 | 06 February 2020 |
| | | | | CA | 3068049 | A1 | 03 January 2019 |
| CN | 101687924 | A | 31 March 2010 | US | 2010240872 | A1 | 23 September 2010 |
| | | | | US | 8546546 | B2 | 01 October 2013 |
| | | | | JPWO | 2009004822 | A1 | 26 August 2010 |
| | | | | JP | 5938816 | B2 | 22 June 2016 |
| | | | | AU | 2008272330 | A1 | 08 January 2009 |
| | | | | EP | 2172483 | A1 | 07 April 2010 |
| | | | | EP | 2172483 | A4 | 04 August 2010 |
| | | | | CA | 2691734 | A1 | 08 January 2009 |
| | | | | WO | 2009004822 | A1 | 08 January 2009 |
| CN | 111836832 | A | 27 October 2020 | AU | 2019205406 | A1 | 27 August 2020 |
| | | | | JP | 2021509896 | A | 08 April 2021 |
| | | | | US | 2020369770 | A1 | 26 November 2020 |
| | | | | KR | 20200118423 | A | 15 October 2020 |
| | | | | TW | 201930349 | A | 01 August 2019 |
| | | | | EP | 3740510 | A1 | 25 November 2020 |

Form PCT/ISA/210 (patent family annex) (July 2022)

## INTERNATIONAL SEARCH REPORT
### Information on patent family members

International application No.

**PCT/CN2024/081748**

| Patent document cited in search report | | | Publication date (day/month/year) | Patent family member(s) | | | Publication date (day/month/year) |
|---|---|---|---|---|---|---|---|
| | | | | EP | 3740510 | A4 | 03 November 2021 |
| | | | | CA | 3085864 | A1 | 11 July 2019 |
| | | | | IL | 275782 | A | 31 August 2020 |
| | | | | SG | 11202006362 | RA | 29 July 2020 |
| | | | | WO | 2019134710 | A1 | 11 July 2019 |
| CN | 115427454 | A | 02 December 2022 | KR | 20220155338 | A | 22 November 2022 |
| | | | | US | 2023129844 | A1 | 27 April 2023 |
| | | | | IL | 296601 | A | 01 November 2022 |
| | | | | BR | 112022018111 | A2 | 25 October 2022 |
| | | | | JP | 2023518225 | A | 28 April 2023 |
| | | | | AU | 2021240028 | A1 | 15 September 2022 |
| | | | | EP | 4121459 | A1 | 25 January 2023 |
| | | | | MX | 2022011632 | A | 13 October 2022 |
| | | | | CA | 3175275 | A1 | 23 September 2021 |
| | | | | WO | 2021188851 | A1 | 23 September 2021 |
| | | | | CL | 2022002526 | A1 | 19 May 2023 |
| CN | 116284408 | A | 23 June 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111500224
- US 8236308 B2
- US 20060286104 A1
- WO 2022060901 A1
- US 20200140552 A1

### Non-patent literature cited in the description

- **SEUNG E ; XING Z ; WU L et al.** A trispecific antibody targeting HER2 and T cells inhibits breast cancer growth via CD4 cells[J. *Nature*, 2022, vol. 603 (7900), 328-334
- **KABAT et al.** *NIH Publ*, 1991, vol. I (91-3242), 647-669
- **SAMBROOK et al.** Molecular Cloning: A Laboratory Manual. Cold Spring Harbor Laboratory Press, 1989
- **LIU R ; OLDHAM R J ; TEAL E et al.** Fc-engineering for modulated effector functions - improving antibodies for cancer treatment[J. *Antibodies*, 2020, vol. 9 (4), 64
- **HA J H ; KIM J E ; KIM Y S.** Immunoglobulin Fc heterodimer platform technology: from design to applications in therapeutic antibodies and proteins [J. *Frontiers in Immunology*, 2016, vol. 7, 394
- **AHMAD Z A ; YEAP S K ; ALI A M et al.** scFv antibody: principles and clinical application[J. *Clinical and developmental immunology*, 2012, vol. 2012, 980250
- **SMITH E ; OLSON K ; HABER L et al.** A novel, native-format bispecific antibody triggering T-cell killing of B-cells is robustly active in mouse tumor models and cynomolgus monkeys[J. *Scientific Reports*, 2016, vol. 5 (1), 17943-17943
- **STEINMETZ A ; VALLÉE F ; BEIL C et al.** CODV-Ig, a universal bispecific tetravalent and multifunctional immunoglobulin format for medical applications [C]//MAbs. *Taylor & Francis*, 2016, vol. 8 (5), 867-878